# EUROPÄISCHE PATENTSCHRIFT

(11) **EP 2 667 384 B1**
(45) Veröffentlichungstag und Bekanntmachung des Hinweises auf die Patenterteilung: **21.12.2016**
(21) Anmeldenummer: 13163067.5
(22) Anmeldetag: 10.04.2013
(51) Int. Cl.: G21K 1/02, G01N 23/20

(54) **Röntgenanalysegerät mit einkristalliner Röntgenblende und Verfahren zur Herstellung einer einkristallinen Röntgenblende**
X-ray analysis device with single crystal x-ray shutter and method for the production of a monocrystalline x-ray shutter
Appareil d'analyse par rayons X avec diaphragme à rayon X monocristallin et procédé de fabrication d'un diaphragme à rayon X monocristallin

(30) Priorität: 24.05.2012 DE 102012208710
(43) Veröffentlichungstag der Anmeldung: 27.11.2013
(73) Patentinhaber: Incoatec GmbH, 21502 Geesthacht (DE)
(72) Erfinder: Kleine, Andreas, 22767 Hamburg (DE); Kreith, Josef, 8700 Leoben (AT); Hertlein, Frank, 23562 Lübeck (DE)
(74) Vertreter: Kohler Schmid Möbus Patentanwälte

(56) Entgegenhaltungen:
- FR-A1- 2 959 344
- JAN SKOV PEDERSEN: "A flux and background optimized version of the NanoSTAR small-angle X-ray scattering camera for solution scattering", JOURNAL OF APPLIED CRYSTALLOGRAPHY, COPENHAGEN, DK, Bd. 37, Nr. 3, 1. Januar 2004 (2004-01-01) , Seiten 369-380, XP009141266, ISSN: 0021-8898
- KHAMSEHPOUR B ET AL: "Drilling of fine apertures in thin metallic foils using a focused ion beam", VACUUM, PERGAMON PRESS, GB, Bd. 44, Nr. 3-4, 1. März 1993 (1993-03-01) , Seiten 361-365, XP025735257, ISSN: 0042-207X, DOI: 10.1016/0042-207X(93)90184-C [gefunden am 1993-03-01]
- NEAL R M ET AL: "POLARIZATION PHASE-SHIFTING POINT-DIFFRACTION INTERFEROMETER", APPLIED OPTICS, OPTICAL SOCIETY OF AMERICA, WASHINGTON, DC; US, Bd. 45, Nr. 15, 20. Mai 2006 (2006-05-20), Seiten 3463-3476, XP001242708, ISSN: 0003-6935, DOI: 10.1364/AO.45.003463

## Beschreibung

Die Erfindung betrifft ein Röntgenanalysegerät, umfassend
- eine Röntgenquelle, von der ein Röntgenstrahl ausgeht,
- wenigstens eine Röntgenblende, die den von der Röntgenquelle ausgehenden Röntgenstrahl begrenzt,
- eine Probe, auf die der von der wenigstens einen Röntgenblende begrenzte Röntgenstrahl gerichtet ist, und
- einen Röntgendetektor zur Detektion einer von der Probe ausgehenden Röntgenstrahlung,
wobei die wenigstens eine Röntgenblende von der Probe beabstandet angeordnet ist.

Eine solches Röntgenanalysegerät ist aus Youli Li et al., J. Appl. Cryst. (2008) 41, 1134-1139 bekannt geworden.

Röntgenmessungen, insbesondere Röntgendiffraktometrie und Röntgenkleinwinkelstreuung, werden zur qualitativen und quantitativen chemischen Analyse sowie zur Strukturanalyse von Proben in verschiedenen Anwendungsgebieten eingesetzt.

In der Röntgendiffraktometrie sind ein hoher Photonenfluss und eine hohe Auflösung gängige Anforderungen an Röntgenanalysegeräte. Hierfür sind neben der Röntgenquelle und der fokussierenden oder kollimierenden Röntgenoptik die Aperturen (Röntgenblenden) von entscheidender Bedeutung.

Auf die Aperturen treffendes Röntgenlicht kann parasitär gestreut werden, was zu einer ungewollten Erhöhung der Untergrundstrahlung und damit zu einer Verschlechterung des Signal-Rausch Verhältnisses führt. Parasitäre Streuung findet einerseits bei polykristallinem Aperturmaterial an den Körnern und Korngrenzen statt und anderseits durch diffuse Streuung an der Oberflächenrauigkeit oder durch Totalreflexion an der Blende selbst.

In Kleinwinkelstreuexperimenten, auch SAXS (=Small Angle X-ray Scattering) genannt, ist die Auflösung bei einer verwendeten Röntgenlichtwellenlänge λ durch den kleinstmöglichen, messbaren Streuwinkel θ bzw. q-Vektor (q=4πsin(θ)/λ) gegeben. Dies bedeutet, dass bei SAXS-Experimenten ein möglichst kleiner Primärstrahlfänger verwendet werden sollte, um eine maximale Auflösung zu garantieren. Da die parasitäre Streuung an den Aperturen bei kleinen Winkeln besonders stark ausgeprägt ist, führt sie zu erhöhten, messbaren Intensitäten um den Primärstrahlfänger und überlagert dadurch Messsignale der Probe, was zu einer Verringerung der Auflösung oder sogar zu einer Beeinträchtigung des korrekten Zählverhaltens des Detektors führen kann.

Des Weiteren erfordern SAXS-Messungen Röntgenstrahlung mit geringer Divergenz. Parasitäre Streuung an den Aperturen vergrößert diese und hat bei Herstellern von Röntgenanalytikgeräten dazu geführt, dass in SAXS-Analytikgeräten jeweils drei Lochblenden mit bestimmter Größe und mit bestimmten Abständen hintereinander in den Strahlengang positioniert werden, um einen Röntgenstrahl mit definierter, niedriger Divergenz zu garantieren. Der Nachteil dieses 3-Lochblenden-Aufbaus besteht zum einen in der massiven Photonenflussreduktion und damit einhergehenden deutlich längeren Messzeiten, und zum anderen in einem großen Platzbedarf dieser Messinstrumente.

In Einkristalldiffraktometrie-Experimenten, auch SCD (=Single crystal diffraction) genannt, werden aufgrund der geringen Probengrößen neben fokussierenden Röntgenoptiken auch Lochblenden, welche in sogenannten Kollimatoren verbaut sind, verwendet. Die Aperturen sind notwendig, um den Röntgenstrahl auf die vorgegebene Probengröße zu begrenzen. Ist der Röntgenstrahl größer als die Probe, erhöht sich das Untergrundrauschen z.B. durch Streustrahlung des Probenhalters und verschlechtert damit das Signal-Rausch-Verhältnis. Die parasitäre Streuung an den Lochblenden führt zu einer weiteren Verschlechterung des Signal-Rausch-Verhältnisses.

Bei SCD- Messungen werden Primärstrahlfänger verwendet, welche möglichst klein zu wählen sind, um den zu untersuchenden q-Raum und damit die Auflösung nur minimal zu limitieren. Wegen der parasitären Streuung an den Lochblenden muss jedoch ein entsprechend größerer Primärstrahlfänger verwendet werden, was zu einer entsprechend geringeren Auflösung führt. Ist die Auflösung um den Primärstrahl entscheidend z.B. bei der Proteinkristallographie, dann können bei gleichbleibender Primärstrahlfängergröße nur sehr kleine Lochblenden verbaut werden, was wiederum den Photonenfluss stark verringert und die Messzeit entsprechend verlängert.

In µ-Diffraktionsanwendungen, auch µ-XRD genannt, soll Röntgenbeugung selektiv nur an sehr kleinen Flächen bzw. Volumina von Proben stattfinden. Dafür muss der Röntgenstrahlquerschnitt auf einen Durchmesser von typischerweise 10-100µm begrenzt werden, wozu neben Röntgenoptiken auch Lochblenden notwendig sind. Die parasitäre Streuung an herkömmlichen, polykristallinen Lochblenden vergrößert dabei die auf der Probe ausgeleuchtete Fläche. Deshalb muss die Lochblendengröße weiter verringert werden, was zu längeren Messzeiten führt. Außerdem haben Messungen gezeigt, dass die polykristalline Struktur der Apertur als Debye-Scherrer-Ringe auf dem Detektor sichtbar wird und dabei das Messsignal der Probe überlagert.

Herkömmliche Röntgenblenden bestehen aus polykristallinen, Röntgenstrahlung sehr gut absorbierenden Metallen wie Wolfram, Tantal, Iridium, Messing, Titan, Molybdän oder Platin, wobei verschiedene Öffnungsgrößen und -Formen angeboten werden. Eine unvorteilhafte Eigenschaft dieser Aperturen ist, dass sie Röntgenstrahlung relativ stark parasitär streuen.

Gehrke et al. in Rev. Sci. Instrum. 66, 1354 (1995) haben eine Röntgenblende für Synchrotronanwendungen vorgeschlagen, mit der die parasitäre Streuung reduziert werden kann. Die Blende umfasst vier motorisierte Schneiden, die einander gegenüberliegend beweglich angebracht sind, so dass eine größenverstellbare, rechteckige Röntgenstrahlquerschnittsfläche entsteht. Die Schneiden bestehen aus einem für Röntgenstrahlung gut absorbierenden Material, an deren Stirnflächen sich einkristalline Siliziumschichten befinden. Zur Vermeidung von Totalreflexion, und damit parasitärer Streuung an den Stirnflächen der Schneiden, werden diese verkippt mit einem Verkippungswinkel größer als der Totalreflexionswinkel.

Ein ähnliches Prinzip ist auch mit Hybrid-Metall-Einkristall-Schneiden bekannt geworden, vgl. Li et al.; Appl. Cryst. 41, 1134 (2008). Anstatt die Schneiden zur Vermeidung von Totalreflexion zu kippen werden jeweils auf der Stirnseite angeschrägte Metallsockel z.B. aus Wolfram oder Messing verwendet, auf die jeweils ein einseitig poliertes, rechteckiges Einkristall-Substrat aufgeklebt wird. Somit wird der Röntgenstrahl hauptsächlich durch die Kanten der einander gegenüberliegenden Einkristall-Substrate definiert, was parasitäre Streuung durch Totalreflexion sowie durch Streuung an Korngrenzen vermeidet.

In der FR 2 955 391 A1 werden Hybrid-Metall-Einkristall-Schneiden in ein Röntgenanalysegerät eingebaut, welches speziell für SAXS-Messungen konzipiert ist und eine kompakte Größe im Vergleich zu anderen kommerziell erhältlichen SAXS-Maschinen haben soll.

Röntgenblenden basierend auf diesen bekannten Hybrid-Metall-Einkristall-Schneiden ermöglichen zwar eine zweidimensionale Strahlformung mit sehr geringer parasitäre Streuung. Sie sind jedoch schwierig zu fertigen; regelmäßig ist der Einsatz von Handarbeit erforderlich. Auch sind diese Röntgenblenden schwierig zu justieren, insbesondere bezüglich der relativen Positionierung der Schneiden. Weiterhin erlauben sie nur einen rechteckigen und hinsichtlich der Auflösung im besten Fall quadratischen Strahlquerschnitt. Die minimale Primärstrahlfängergröße wird durch den Abstand vom Mittelpunkt zum äußersten Punkt der Strahlquerschnittsfläche gegeben. Dies bedeutet, dass ein runder Strahlquerschnitt die kleinste Primärstrahlfängergröße und damit die höchste Auflösung erlaubt. Im Vergleich dazu ist die Auflösung eines quadratischen Strahlquerschnitts und damit auch die der Systeme mit vier Hybrid-Schneiden um mindestens 41.4% (entspricht √2) schlechter.

Aus der JP 2002 250 704 A ist eine Maske bekannt geworden, die an eine Probe angelegt wird, um einen Teil der Probe für eine Belichtung mit Röntgenstrahlung auszuwählen. Die Maske ist mit einem trichterförmigen Durchbruch ausgebildet, wobei der engere Teil des Durchbruchs der Probe zugewandt ist. Die Maske ist aus einem einkristallinen Material gefertigt.

### Aufgabe der vorliegenden Erfindung

Es ist die Aufgabe der vorliegenden Erfindung, ein Röntgenanalysegerät vorzustellen, bei dem bei geringem Aufwand Röntgenmessungen durch parasitäre Streustrahlung weniger beeinträchtigt werden.

### Kurze Beschreibung der Erfindung

Diese Aufgabe wird gelöst durch ein Röntgenanalysegerät der eingangs genannten Art, das dadurch gekennzeichnet ist,
dass die wenigstens eine Röntgenblende einen einkristallinen Blendenkörper mit einem durchgehenden Loch umfasst,
wobei der einkristalline Blendenkörper eine umlaufende, durchgehende, den Röntgenstrahl begrenzende Kante ausbildet, von der ausgehend sich das Loch in einem ersten Bereich in Richtung einer Austrittsöffnung der Röntgenblende trichterförmig erweitert.

Gemäß der Erfindung wird die Röntgenblende durch einen einstückigen Einkristall ausgebildet, welcher mit einem Loch versehen ist, durch den Röntgenstrahlung dringen kann. Das Loch weist eine Kante auf, durch die die Röntgenstrahlung abgeschattet wird. Von der Kante weg in Strahlausbreitungsrichtung weitet sich das Loch auf, wodurch zum einen eine scharfe (bevorzugt atomar scharfe) Kante ausgebildet werden kann; zum anderen können Totalreflexionen an der Röntgenblende jenseits der Kante vermieden werden.

Die Blende benötigt keine beweglichen Schneiden, wodurch die Blende einfach zu fertigen und zu handhaben ist. Insbesondere ist die Fertigung von Blenden mit einkristallinem Blendenkörper für die Erfindung maschinell (und daher kostengünstig) mit hoher Reproduzierbarkeit möglich.

Durch das einkristalline Blendenmaterial wird eine besonders geringe parasitäre Streuung erreicht; Streuung an Körnern oder Korngrenzen findet nicht statt. Entsprechend kann ein verbessertes Signal-zu-Rauschverhältnis erreicht werden. Durch ein erfindungsgemäßes Herstellungsverfahren (siehe unten) können weiterhin Defekte und Rauigkeit im Bereich der Kante minimiert werden, wodurch eine praktisch streufreie Röntgenblende (Apertur) verfügbar wird.

Die Anzahl der Lochblenden in einem SAXS-Aufbau kann im Rahmen der Erfindung auf ein oder zwei reduziert werden. Hierdurch sind höhere Photonenflüsse und kleinere Primärstrahlfänger möglich, was wiederum die Auflösung erhöht und die Messzeiten verkleinert. Außerdem ist die Strahldivergenz über den Abstand der Lochblenden einfach einstellbar und kann gegebenenfalls reduziert werden. Des Weiteren wird der Platzbedarf eines SAXS-Messinstruments stark reduziert.

In einem SCD-Aufbau können im Rahmen der Erfindung bei gleichem Photonenfluss deutlich kleinere Primärstrahlfänger verwendet werden als im Stand der Technik, was zu einer erheblichen Vergrößerung der Auflösung führt. Bleibt die Primärstrahlfängergröße unverändert, können größere Lochblendendurchmesser verwendet werden, was höhere Photonenflüsse und damit kürzere Messzeiten ermöglicht. Außerdem kann die Anzahl der Lochblenden auf ein oder zwei reduziert werden, was den Platzbedarf verkleinert.

In µ-XRD Messanordnungen können größere Lochdurchmesser verwendet werden, was zu höheren Photonenflüssen und damit kürzeren Messzeiten führt. Außerdem überlagern nicht mehr Debye-Scherrer-Ringe von einem polykristallinen Lochblendenmaterial das zu messende Signal der Probe. Das einkristalline Blendenmaterial erzeugt nur einzelne Beugungsreflexe am Detektor. Diese Reflexe überlagern erstens deutlich weniger das Messsignal der Probe und zweitens können sie eindeutig den Lochblenden zu geordnet werden, was eine Auswertung der Messergebnisse vereinfacht.

Das Loch kann mit einer den Erfordernissen des gewählten Röntgenanalyseexperiments angepassten Form gefertigt werden. Die Form des Lochs ist nicht auf eine rechteckige Form begrenzt, sondern kann grundsätzlich beliebig gewählt werden. Insbesondere kann eine kreisrunde Form gewählt werden, mit der ein besonders kleiner, bevorzugt kreisrunder Primärstrahlfänger eingesetzt werden kann, was das Auflösungsvermögen gegenüber einem quadratischen Strahlquerschnitt in SAXS- und SCD-Messungen um einen Faktor √2 verbessert. Das Loch bzw. die Strahlform kann insbesondere auch an die Probengeometrie angepasst werden, etwa um eine gleichmäßige Ausleuchtung der Probe sicherzustellen.

Weiterhin können die streufreien (oder zumindest streuarmen) Röntgenblenden mit einkristallinem Blendenkörper im Vergleich zu herkömmlichen (polykristallinen) Röntgenblenden viel schneller in den Strahlengang eines Röntgenanalysegeräts einjustiert werden, da die verringerte parasitäre Streuung größere Toleranzen erlaubt. In Ermangelung von beweglichen Schneiden entfällt auch eine relative Justage dieser Schneiden.

Beim Umrüsten eines bestehenden SAXS-Laborgerätes sind kompakte, streufreie (oder zumindest streuarme) Röntgenblenden mit einkristallinem Blendenkörper gemäß der Erfindung gegenüber bekannten Schneide-Systemen deutlich zu bevorzugen, weil ein Aperturwechsel mit den Röntgenblenden gemäß der Erfindung schneller, leichter und billiger ist und obendrein eine höhere Auflösung garantiert.

Das Material des einkristallinen Blendenkörpers kann insbesondere Silizium oder Germanium sein; diese Materialien sind kostengünstig in hoher Reinheit einkristallin verfügbar. Weitere Blendenkörpermaterialien können beispielsweise einkristallines Tantal, Platin, Wolfram, Blei oder Iridium sein. Letztere Materialien verfügen über eine besonders hohe Dichte und zeigen daher ein größeres Absorptionsvermögen; sie sind für hochenergetische Röntgenstrahlung bzw. für Röntgenstrahlung hoher Intensität bevorzugt. Bei der Materialauswahl sollte aber darauf geachtet werden, dass die eingestrahlte Röntgenstrahlung keine Fluoreszenzstrahlung und/oder Braggreflexe im kleinen Winkelbereich im Einkristall anregt, was zum parasitären Streusignal beitragen könnte. Der Blendenkörper kann insbesondere aus einem Wafer, etwa einem Standardwafer (soweit verfügbar), bevorzugt einseitig oder zweiseitig poliert zur Reduktion der Oberflächenrauigkeit, ausgeschnitten sein. Das Blendenkörpermaterial kann dotiert sein; bevorzugt wird jedoch undotiertes Material mit einer möglichst kleinen Defektdichte eingesetzt.

Die Blendendicke des Blendenkörpers (Abstand Eintrittsöffnung zu Austrittsöffnung, in Strahlrichtung) sollte wenigstens 0,05 mm betragen und liegt bevorzugt in einem Bereich von 0,1 mm bis 3 mm.

Das Loch der Röntgenblende hat im Bereich der Kante seine minimale Querschnittsfläche, welche die strahldefinierende Querschnittsfläche ist. In Strahlrichtung vor und hinter der Kante tritt das Material des Blendenkörpers quer zur Strahlrichtung nach außen zurück, etwa trichterförmig oder auch als senkrecht zur Strahlrichtung verlaufende Wand. Typischerweise ist die Röntgenblende im Bereich ihres Lochs achsensymmetrisch, insbesondere rotationssymmetrisch zu einer Symmetrieachse aufgebaut, welche durch das Zentrum der strahldefinierenden Querschnittsfläche verläuft; dies vereinfacht die Fertigung und garantiert eine optimale Strahlformung bei geringer parasitärer Streuung.

Bei erfindungsgemäßen Röntgenanalysegeräten befindet sich der Strahlengang bevorzugt ganz oder teilweise in einem evakuierten Volumen bei einem Druck zwischen 10 mbar und 10⁻⁸ mbar. Der Strahlengang kann auch ganz oder teilweise innerhalb eines Schutzgases wie z.B. Helium oder Stickstoff verlaufen. Beide Varianten vermindern bzw. verhindern Luftstreuung, was das Signal-zu-Rauschverhältnis verbessert.

### BevorzugteAusführungsformen der Erfindung

Bei einer bevorzugten Ausführungsform des erfindungsgemäßen Röntgenanalysegeräts weist das Loch zumindest im Bereich der Kante und des ersten Bereichs einen runden, insbesondere kreisrunden Querschnitt auf. In diesem Fall kann auch ein runder, insbesondere kreisrunder Primärstrahlfänger eingesetzt werden, wodurch eine minimale Primärstrahlfängergröße gewählt werden kann. Dadurch sind besonders hohe Auflösungen möglich; mit einer kreisrunden Lochform bzw. einem kreisrundem Röntgenstrahlquerschnitt wird eine Verbesserung um einen Faktor √2 gegenüber einer quadratischen Blende bzw. einem quadratischen Strahlquerschnitt erreicht. Besonders bevorzugt ist ein Durchmesser der (kreisrunden) strahldefinierenden Querschnittsfläche in einem Bereich von 0,001 mm bis 3 mm, insbesondere von 0.05 mm bis 1.5mm. Alternativ können beispielsweise auch rechteckige Blendenquerschnitte eingesetzt werden.

Besonders bevorzugt ist eine Ausführungsform, bei der die Kante in einer Kantenebene verläuft, wobei eine Flächennormale der Kantenebene im Wesentlichen parallel zur Ausbreitungsrichtung des von der Röntgenblende begrenzten Röntgenstrahls verläuft. Dadurch kann insgesamt ein günstiges Streuverhalten eingerichtet werden.

Bevorzugt ist weiterhin eine Ausführungsform, bei der der erste Bereich kegelstumpfartig ausgebildet ist und einen Öffnungswinkel α zwischen 5° und 60°, bevorzugt zwischen 10° und 30°, besonders bevorzugt zwischen 12° und 18° aufweist. Diese Öffnungswinkel haben sich in der Praxis bewährt. Sie sind insbesondere bei den bevorzugten Materialien des Blendenkörpers (etwa Si, Ge oder Ta) bei den typischen Röntgenwellenlängen (etwa Cu-Kα-Strahlung) für die Vermeidung einer Totalreflexion im ersten Bereich ausreichend groß und gut zu fertigen. Der Öffnungswinkel α ist der Neigungswinkel der Mantelfläche des (geraden Kreis-)Kegelstumpfs, also der Lochwand im ersten Bereich, gegen die (Rotationssymmetrie-)Achse des (geraden Kreis-)Kegelstumpfs, die typischerweise der Ausbreitungsrichtung des von der Röntgenblende begrenzten Röntgenstrahls entspricht.

Besonders vorteilhaft ist auch eine Ausführungsform, bei der ausgehend von der Kante sich das Loch in einem zweiten Bereich in Richtung einer Eintrittsöffnung der Röntgenblende trichterförmig erweitert. Im Vergleich zu einer einseitigen trichterförmigen Erweiterung befindet sich hier mehr einkristallines Material in unmittelbarer Nähe der Kante. Dadurch wird eine höhere Absorption der auf die Apertur (und nicht auf das Loch) treffenden Röntgenstrahlung erreicht.

Eine bevorzugte Weiterbildung dieser Ausführungsform sieht vor, dass der zweite Bereich kegelstumpfartig ausgebildet ist und einen Öffnungswinkel β zwischen 20° und 80°, bevorzugt zwischen 25° und 70°, besonders bevorzugt zwischen 30° und 60° aufweist. Dies hat sich wiederum in der Praxis bewährt, insbesondere für eine einfache Fertigung und zur Vermeidung von Totalreflexionen. Der Öffnungswinkel β ist der Neigungswinkel der Mantelfläche des (geraden Kreis-)Kegelstumpfs, also der Lochwand im zweiten Bereich, gegen die (Rotationssymmetrie-)Achse des (geraden Kreis-)Kegelstumpfs, die typischerweise der Ausbreitungsrichtung des von der Röntgenblende begrenzten Röntgenstrahls entspricht.

Bei einer alternativen Ausführungsform ist die Kante an einer Vorderseite der Röntgenblende ausgebildet, so dass die Kante eine Eintrittsöffnung der Röntgenblende mit begrenzt. Mit anderen Worten, die Kante befindet sich direkt an der quellzugewandten Vorderseite des Blendenkörpers. Diese Ausführungsform ist besonders einfach zu fertigen.

Erfindungsgemäß ist vorgesehen, dass bei der Röntgenblende in einem Wellenlängenbereich von 10nm bis 1µm die Rautiefe zumindest im Bereich der Kante kleiner als 100nm ist. Bei dieser Rauigkeit ist die parasitäre Streuung besonders gering; die geforderte Rauigkeit - hier in Gestalt einer Rautiefe (peak-to-valley) - kann mit dem unten beschriebenen, erfindungsgemäßen Herstellungsverfahren erreicht werden. Typischerweise gilt die gleiche Rauigkeitsbedingung auch im ersten Bereich und ggf. im zweiten Bereich.

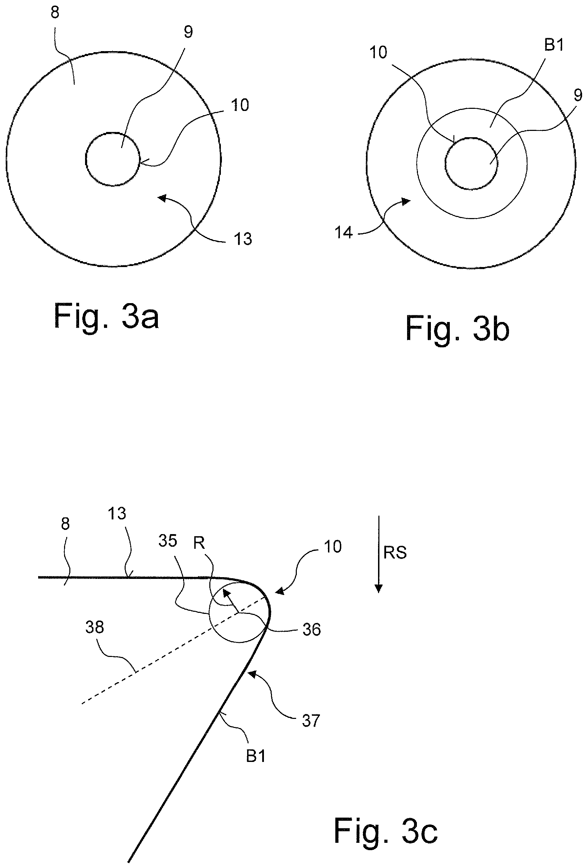

Ebenfalls bevorzugt ist eine Ausführungsform, bei der für eine Kantenschärfe der Kante der Röntgenblende gilt: Kantenradius < 5µm. Der Kantenradius ist der Radius des größten Kreises, der innen an das Profil der Kante (mit der Kreisebene parallel zur Symmetrieachse des Lochs bzw. parallel zur Strahlrichtung) angelegt werden kann, ohne das Profil zu schneiden (siehe dazu auch Fig. 3c); das Profil der Kante kann beispielsweise im Rasterelektronenmikroskop nach einer entsprechenden Präparation des Blendenkörpers (etwa Aufsägen oder Aufpolieren) ersehen werden. Ein Kantenradius < 5µm trägt dazu bei, die parasitäre Streuung gering zu halten; die geforderte Kantenschärfe kann ebenfalls mit dem erfindungsgemäßen Herstellungsverfahren eingerichtet werden.

Ebenso bevorzugt ist eine Ausführungsform, bei der die Röntgenblende so ausgebildet ist, dass bei Bestrahlung der Kante mit einem näherungsweise parallelen Röntgenstrahl bei halber Blendenüberdeckung ein integraler Streulichtanteil, welcher ab einem Streuwinkel von 1 mrad gemessen wird, kleiner als 5*10⁻⁵ ist. Die geforderte, geringe parasitäre Streuung kann mit einer gemäß dem unten vorgestellten Herstellungsverfahren gefertigter Röntgenblende erreicht werden; mit einer solchen Blende bzw. einem zugehörigen Röntgenanalysegerät können besonders hochauflösende Röntgenmessungen durchgeführt werden.

Besonders vorteilhaft ist auch eine Ausführungsform, die vorsieht, dass der einkristalline Blendenkörper mit einer Absorptionsstruktur verbunden, insbesondere verklebt, ist, dass das Material der Absorptionsstruktur Röntgenstrahlung stärker absorbiert als das Material des einkristallinen Blendenkörpers, insbesondere wobei das Material der Absorptionsstruktur Wolfram, Tantal oder Blei ist, und dass die Absorptionsstruktur quer zur Ausbreitungsrichtung des von der wenigstens einen Röntgenblende begrenzten Röntgenstrahls gegenüber der Kante zurückgesetzt ist. Durch die Absorptionsstruktur wird eine Durchstrahlung der Blende mit Röntgenlicht entfernt vom Loch erschwert, allein schon aufgrund des verlängerten Absorptionswegs. Das Material der Absorptionsstruktur ist bevorzugt einkristallin (es muss aber nicht einkristallin sein); insbesondere kommen Pt, Ir, Ta, Pb, Mo, Ti, Ag und W als Materialien in Betracht. Parasitäre Streuung an der Absorptionsstruktur spielt praktisch keine Rolle, da der einkristalline Blendenkörper die Absorptionsstruktur zum Loch hin in einem der Röntgenquelle (Wellenlänge und Intensität) angepassten Abstand (typischerweise überall an der umlaufenden Kante um wenigstens 0,1 mm) überragt und daher Streustrahlung abblockt. Absorptionsstrukturen werden insbesondere bei hoher Intensität der Röntgenstrahlung der Röntgenquelle oder auch hoher Energie (kurze Wellenlänge) der Röntgenstrahlung eingesetzt. Absorptionsstrukturen können beispielsweise an einer (bezüglich der Strahlausbreitungsrichtung) Vorderwand, einer Hinterwand, einer Tunnelinnenwand (Lochinnenseite, Trichterwand) und/oder in einer Sandwichanordnung an der Röntgenblende vorgesehen sein.

Absorptionsstrukturen oder deren Teile können beispielsweise in Form von Folien oder Platten mit Loch für den Röntgenstrahl oder als abgeschiedene Schichten auf dem (ohnehin mit Loch versehenen) Blendenkörper realisiert sein. Die strahldefinierende Querschnittsfläche der Blende wird von einer Absorptionsstruktur aber weder vollständig noch teilweise verdeckt.

Vorteilhaft ist eine Weiterbildung dieser Ausführungsform, bei der die Absorptionsstruktur eine Halterung ausbildet, die quer zur Ausbreitungsrichtung des von der wenigstens einen Röntgenblende begrenzten Röntgenstrahls über den Blendenkörper herausragt. Die Halterung ermöglicht einen leichten und schnellen Einbau der Röntgenblende in ein erfindungsgemäßes Röntgenanalysegerät; ggf. ist auch die Handhabung oder Befestigung während eines Nachbearbeitungsprozesses erleichtert. Die Halterung kann beispielsweise Gewinde, Haken, Rillen, Aussparungen, Ausbuchtungen, Vertiefungen, Löcher oder dergleichen für eine Befestigung der Absorptionsstruktur umfassen.

Eine vorteilhafte Ausführungsform sieht vor, dass der einkristalline Blendenkörper mit einer Abdeckstruktur verbunden, insbesondere verklebt, ist, dass das Material der Abdeckstruktur Röntgenstrahlung schwächer absorbiert als das Material des Blendenkörpers, insbesondere wobei das Material der Abdeckstruktur Kapton oder Beryllium ist, und dass die Abdeckstruktur eine Eintrittsöffnung und/oder die Austrittsöffnung der Röntgenblende überdeckt, insbesondere wobei das Loch durch die Abdeckstruktur gasdicht oder vakuumdicht verschlossen ist. Die Abdeckstruktur kann zum einen den Bereich der Kante vor Verschmutzungen schützen, insbesondere bei einem beidseitigen Verschluss. Zum anderen kann die Abdeckstruktur - abhängig von ihrem Material - als Filter für elektromagnetische Strahlung bestimmter Energiebereiche (bevorzugt innerhalb des Energiebereichs von 100 eV bis 100 keV) dienen. Schließlich kann im Falle eines gasdichten oder vakuumdichten Verschlusses des Lochs (der einseitig oder auch beidseitig sein kann) die Röntgenblende als Kombinationsbauteil zusätzlich als Röntgenfenster dienen. In letzterem Fall ist das Material der Abdeckstruktur im Bereich 100 eV bis 100 keV bevorzugt nur schwach streuend und schwach absorbierend. Bereiche verschiedenen Drucks sind in Röntgenanalysegeräten häufig anzutreffen, so dass mit dem Kombinationsbauteil (mit dem diese Bereiche getrennt werden können) ein kompakterer Aufbau erreicht werden kann. Soweit ein Vakuum abgetrennt werden soll, ist die Abdeckstruktur vorzugsweise bis 10⁻⁸ mbar vakuumdicht. Das Material der Abdeckstruktur ist bevorzugt folienförmig oder plattenförmig, typischerweise mit einer Dicke im Bereich 5 µm bis 1 mm. Die Abdeckstruktur ist typischerweise an der Vorderseite und/oder der Rückseite der Röntgenblende angeordnet.

Ebenfalls vorteilhaft ist eine Ausführungsform, die vorsieht, dass der einkristalline Blendenkörper mit einer Füllstruktur verbunden, insbesondere verklebt, ist, dass das Material der Füllstruktur Röntgenstrahlung schwächer absorbiert als das Material des Blendenkörpers, insbesondere wobei das Material der Füllstruktur Kapton oder Beryllium ist, und dass die Füllstruktur das Loch ausfüllt. Die Füllstruktur kann das Loch effektiv vor Verschmutzung und mechanischer Beschädigung schützen. Ebenso kann das Material der Füllstruktur als Energiefilter dienen. Darüber hinaus kann die Füllstruktur, wenn sie das Loch gleichzeitig gasdicht oder vakuumdicht verschließt, die Röntgenblende zu einem Kombinationsbauteil machen, das auch als Röntgenfenster eingesetzt werden kann. Details zu diesen Funktionen sind bei der vorhergehenden Ausführungsform beschrieben, die für diese Ausführungsform ebenfalls zutreffen.

Bevorzugt ist auch eine Ausführungsform, bei der das Röntgenanalysegerät zwei von der Probe beabstandet angeordnete Röntgenblenden umfasst, die jeweils einen einkristallinen Blendenkörper mit einem durchgehenden Loch umfassen, wobei die einkristallinen Blendenkörper jeweils eine umlaufende, durchgehende, den Röntgenstrahl begrenzende Kante ausbilden, von der ausgehend sich das Loch des jeweiligen Blendenkörpers in einem ersten Bereich in Richtung einer Austrittsöffnung der jeweiligen Röntgenblende trichterförmig erweitert. In vielen Anwendungsfällen, insbesondere für eine SAXS-Apparatur, sind genau zwei Röntgenblenden mit einkristallinem Blendenkörper ausreichend (anstelle von drei herkömmlichen Blenden), wodurch ein höherer Photonenfluss - und damit geringere Messzeit - und ein kompakterer Bau erreicht werden können; die parasitäre Streuung ist zudem vermindert, so dass auch eine höhere Auflösung erzielt werden kann. Entsprechend den oben dargestellten Ausführungsformen können jeweils eine oder beide der Röntgenblenden ausgebildet sein. Beide Röntgenblenden sind (bezogen auf die Ausbreitungsrichtung des Röntgenstrahls) vor der Probe angeordnet. Die Röntgenblenden sind typischerweise mit kegelstumpfartigen ersten Bereichen versehen, deren Rotationssymmetrieachsen auf derselben Geraden liegen, wobei die Gerade parallel zur Ausbreitungsrichtung des Röntgenstrahls verläuft. Bevorzugt ist der Abstand zwischen den beiden Blenden variabel einstellbar; dadurch kann die Divergenz eingestellt und auf das durchzuführende Experiment angepasst werden. Die Divergenz div kann abgeschätzt werden über div=(d1+d2)/l12, mit d1, d2: Durchmesser der strahlformenden Querschnittsflächen der Blenden 1, 2, und I12: Abstand der Blenden 1, 2. Bei klein eingestellter Divergenz (also großem Blendenabstand I12 bzw. kleinem d1, d2) kann die Größe des Primärstahlfängers entsprechend klein gewählt werden, so dass ein erhöhtes Auflösungsvermögen erreicht wird.

Vorteilhafter Weise ist in einer Ausführungsform des erfindungsgemäßen Röntgenanalysegeräts vorgesehen, dass das Röntgenanalysegerät ein strahlformendes Element, insbesondere einen Göbelspiegel oder Montelspiegel, aufweist, das direkt an der Röntgenquelle angeordnet ist. Mit dem strahlformenden Element wird der von der Quelle ausgehende Röntgenstrahl typischerweise parallelisiert oder fokussiert (etwa auf die Probe); auch ist eine zusätzliche Monochromatisierung möglich (falls erforderlich). Am strahlformenden Element kann wiederum ausgangsseitig eine Röntgenblende gemäß der Erfindung befestigt sein. Diese Ausführungsform trägt zu einem kompakten Aufbau bei. Man beachte, dass gemäß der Erfindung ein strahlformendes Element auch unabhängig (beabstandet) von der Röntgenquelle platziert werden kann. Ebenso ist es möglich, eine Röntgenblende gemäß der Erfindung an einer Vakuumkammer/Schutzgaskammer oder als Teil der äußeren Begrenzung einer Vakuumkammer/Schutzgaskammer anzuordnen, oder eine Röntgenblende gemäß der Erfindung solitär anzuordnen. Außerdem ist es möglich, eine oder mehrere Röntgenblenden gemäß der Erfindung in einem Kollimator, wie er z.B. bei SCD- oder µ-XRD-Experimenten verwendet wird, zu verbauen und in das erfindungsgemäße Röntgenanalysegerät zwischen Röntgenquelle und Probe bzw. zwischen Röntgenoptik (strahlformendem Element) und Probe zu positionieren.

Besonders bevorzugt ist auch eine Ausführungsform, bei der das Röntgenanalysegerät als eine Kleinwinkelstreuungs-Messanordnung oder eine Einkristallstreuungs-Messanordnung ausgebildet ist, insbesondere mit einem Primärstrahlfänger. Für eine Kleinwinkelstreuungs-Messanordnung (SAXS-Messanordnungen; SAXS= small angle x-ray scattering) werden nur ein bis zwei Röntgenblenden mit einkristallinem Blendenkörper benötigt, mit dem Vorteil eines höheren Photonenflusses, eines kompakteren Baus und einer höheren Auflösung. Ähnliche Vorteile ergeben sich auch bei Einkristallstreuungs-Messanordnungen bzw. Einkristalldiffraktometrie-Messanordnungen (auch SCD-Messanordnungen genannt; SCD= single crystal diffraction, Einkristallbeugung). Ähnlich wie bei SAXS-Messungen bedingen in der SCD-Anwendung herkömmliche (polykristalline) Blenden entweder einen sehr großen Primärstrahlfänger, was die Auflösung herunter setzt, oder aber es werden sehr kleine Lochblenden eingebaut, um entsprechend kleine Primärstrahlfänger verwenden zu können. In letzterem Fall wird jedoch die Intensität des Röntgenstrahls stark reduziert, was zu längeren Messzeiten führt. Durch den Einsatz von streuarmen bzw. streufreien Blenden mit einkristallinem Blendenkörper kann in SCD -Aufbauten der standardmäßig verwendete, 1,5 mm große Primärstrahlfänger deutlich verkleinert werden, etwa auf einen Durchmesser von 1 mm oder weniger, was ein deutlich verbessertes Auflösungsvermögen ermöglicht.

Eine weitere bevorzugte Ausführungsform des erfindungsgemäßen Röntgenanalysegerätes sieht vor, dass das Röntgenanalysegerät als eine µ-XRD-Messanordnung ausgebildet ist. Typischerweise haben in dieser Messanordnung die Lochblenden (mit einkristallinem Blendenkörper) einen Durchmesser in einem Bereich von 10µm bis 100µm. Da bei streufreien bzw. streuarmen Lochblenden (fast) keine parasitäre Streuung erzeugt wird, können, verglichen mit herkömmlichen, polykristallinen Aperturen, größere Lochdurchmesser verwendet werden, was den Photonenfluss erhöht und die Messzeit verringert. Außerdem erzeugen die einkristallinen Lochblenden keine das Messsignal überlagernden Debye-Scherrer-Ringe auf dem Detektor, sondern nur einzelne, eindeutig zu zuordnende Beugungsreflexe. Diese Eigenschaft vereinfacht die Auswertung der Messergebnisse.

### Erfindungsgemäßes Herstellungsverfahren und Nachbearbeitungsverfahren

In den Rahmen der vorliegenden Erfindung fällt auch ein Verfahren zur Herstellung einer Röntgenblende, mit folgenden Schritten:
a) Einbringen eines durchgehenden Lochs in einen einkristallinen
   Blendenkörper, wobei zum Einbringen des Loches Funkenerosion und/oder kalte Laserablation eingesetzt wird, so dass der einkristalline Blendenkörper eine umlaufende, durchgehende Kante zur Begrenzung eines Röntgenstrahls ausbildet, von der ausgehend sich das Loch in einem ersten Bereich in Richtung einer Austrittsöffnung trichterförmig erweitert,
b) Abtragen von Material von der Oberfläche des Blendenkörpers zumindest im Bereich der Kante und des ersten Bereichs durch lonenstrahlätzen.

Im Rahmen des erfindungsgemäßen Verfahrens kann auf einfache Weise eine streufreie oder zumindest streuarme Röntgenblende für eine obige, erfindungsgemäße Röntgenanalyseapparatur hergestellt werden.

Bei der erfindungsgemäßen Nachbearbeitung gemäß Schritt b) werden Ionen, bevorzugt Argon-lonen, in einem Vakuum beschleunigt, typischerweise mit einer Beschleunigungsspannung im Bereich 1 kV bis 10 kV, und mit hoher kinetischer Energie auf die Oberfläche des einkristallinen Blendenkörpers gelenkt, insbesondere im Bereich der Kante. Die beschleunigten Ionen tragen dabei Material von der Oberfläche des einkristallinen Blendenkörpers ab (etwa oberflächige Verschmutzungen, aber auch das einkristalline Material des Blendenkörpers selbst). Im Rahmen von Schritt b) wird aber typischerweise nur eine geringe Menge an Material im Bereich einiger zehn µm (etwa 10 µm bis zu 100µm) abgetragen; bei einem kreisrunden Loch vergrößert sich dessen Durchmesser durch die Nachbehandlung gemäß Schritt b) typischerweise um ca. 100 µm (was erforderlichenfalls in Schritt a) berücksichtigt werden sollte). Oberflächennahe Defekte, die im Rahmen von Schritt a) in den Einkristall im Bereich des Lochs bzw. der Kante eingetragen wurden, werden abgetragen; effektiv wird dadurch die Defektdichte im Blendenkörper verringert, was die parasitäre Streuung verringert.

Mit dem Ionenätzprozess ist eine sorgfältige Nachbearbeitung der Kante bzw. deren Umgebung möglich; dies ist besonders wichtig, da hier durch das erfindungsgemäße Design wenig absorbierendes, einkristallines Material vorhanden ist und andererseits hier besonders viele Photonen auf die Apertur treffen (wenn man etwa von einem gaußförmigen Intensitätsprofil des Röntgenstrahls ausgeht) und parasitär gestreut werden können. Außerdem werden Absorbate und Adsorbate entfernt sowie die Oberflächenrauigkeit reduziert; auch diese beiden Effekte reduzieren die parasitäre Streuung im späteren Einsatz in einem Röntgenanalysegerät.

Bevorzugt wird im Rahmen von Schritt b) die gesamte Lochoberfläche des Blendenkörpers nachbearbeitet; falls ein trichterförmiger zweiter Bereich zur Seite der Eintrittsöffnung hin vorgesehen ist, wird dieser bevorzugt auch nachbearbeitet. Vorteilhafter Weise kann der Auftreffwinkel der Ionen auf die Oberfläche des Blendenkörpers variiert werden, etwa durch Verkippen der lonenquelle oder des Blendenkörpers. Vorteilhafter Weise kann die lonenquelle auch relativ zum Blendenkörper verfahren werden, etwa um den Abstand zu variieren (zur Einstellung der Abtragrate und/oder des Polierquerschnitts) oder auch um die vom lonenstrahl überstrichene Fläche zu verschieben (etwa wenn der Durchmesser des lonenstrahls zur gleichzeitigen Bearbeitung des gesamten relevanten Teils des Blendenkörpers nicht ausreicht). Typischerweise ist der lonenstrahl so eingestellt, dass er parallel zur Symmetrieachse der Blende (was auch der vorgesehenen Röntgenstrahlrichtung entspricht) ausgerichtet ist.

Gemäß des erfindungsgemäßen Herstellungsverfahrens wird in Schritt a) zum Einbringen des Loches Herstellungsverfahrens wird in Schritt a) zum Einbringen des Loches Funkenerosion und/oder kalte Laserablation eingesetzt. Im Rahmen von kalter Laserablation wird bevorzugt ein gepulster Laserbetrieb mit einer Impulsdauer eines einzelnen Laserimpulses von 15 ps oder weniger eingesetzt. Beide Verfahren (Funkenerosion und kalte Laserablation) haben sich zur Einbringung von (kreis-)runden Löchern gut bewährt. Bei Anwendung dieser Verfahren werden nur geringfügige (insbesondere oberflächennahe) Defekte in den einkristallinen Blendenkörper eingebracht. Im Gegensatz dazu hat sich beispielsweise mechanisches Bohren im Rahmen von Schritt a) nicht bewährt, da hierbei auch Defekte in tieferen Schichten des Blendenkörpers entstehen. Ebenfalls von Bedeutung ist, dass mit kalter Laserablation und Funkenerosion vergleichsweise große Öffnungswinkel α des ersten Bereichs (etwa ≥ 15°) im Rahmen von Schritt a) eingebracht werden können, die beispielsweise mit DRIE (deep reactive ion etching, tiefes reaktives lonenätzen) nicht oder nur sehr schwer realisierbar sind.

Bevorzugt ist eine Variante, bei der das Loch in Schritt a) zumindest im Bereich der Kante und des ersten Bereichs mit einem runden, insbesondere kreisrunden Querschnitt gefertigt wird. Der (kreis-)runde Querschnitt bleibt bei der Nachbearbeitung in Schritt b) erhalten, so dass die gefertigte Röntgenblende dann in einem Röntgenanalysegerät mit hohem Auflösungsvermögen bzw. mit einem besonders kleinen Primärstrahlfänger eingesetzt werden kann.

Vorteilhaft ist eine Weiterentwicklung dieser Verfahrensvariante, bei der in Schritt b) ein lonenstrahl mit kreisrundem Querschnitt und gaußverteilter Intensität eingesetzt wird, dessen Intensitätsmaximum auf einer Symmetrieachse der strahldefinierenden Querschnittsfläche des Lochs des einkristallinen Blendenkörpers liegt. Dadurch wird auf einfache Weise verhindert, dass die (kreis-)runde Öffnung bzw. das Loch im Blendenkörper durch die Nachbearbeitung die Form ändert. Der lonenstrahl, der entlang der Symmetrieachse eingestrahlt wird, hat bevorzugt einen Strahldurchmesser (gemäß FWHM, full width at half maximum) von wenigstens der Hälfte des gewünschten Durchmessers der strahldefinierenden Querschnittsfläche des Lochs.

Besonders bevorzugt ist eine Variante, bei der zwischen Schritt a) und Schritt b) eine Reinigung des Blendenkörpers in einem Ultraschallbad erfolgt. Dadurch können grobe Verschmutzungen an der Oberfläche verringert werden, die durch lonenstrahlätzen in Schritt b) nicht oder nur schwer zu entfernen wären. Somit trägt die Ultraschallbadreinigung ebenfalls zur Reduktion von parasitärer Streuung bei.

Eine vorteilhafte Verfahrensvariante sieht vor, in Schritt b) eine einer lonenquelle abgewandte Öffnung, nämlich eine Eintrittsöffnung oder die Austrittsöffnung der Röntgenblende, freigehalten wird. Durch die freigehaltene Öffnung kann abgetragenes Material entweichen, was die Effektivität der Reinigungswirkung des lonenstrahlätzens verbessert.

Bevorzugt ist auch eine Verfahrensvariante, bei der in Schritt b) Ionen ausschließlich auf den einkristallinen Blendenkörper gerichtet sind. Insbesondere werden keine Ionen auf Absorptionsstrukturen gerichtet; jedoch darf der Ionenstrahl das Loch teilweise oder ganz durchstrahlen. Dadurch wird eine Verunreinigung der Kante durch Fremdmaterial (etwa aus der Absorptionsstruktur) vermieden.

Bei einer vorteilhaften Verfahrensvariante ist vorgesehen, dass in Schritt a) das Loch in den einkristallinen Blendenkörper so eingebracht wird, dass ausgehend von der Kante sich das Loch in einem zweiten Bereich in Richtung einer Eintrittsöffnung der Röntgenblende trichterförmig erweitert, und dass in Schritt b) auch im zweiten Bereich Material von der Oberfläche des Blendenkörpers durch lonenstrahlätzen abgetragen wird. Im Bereich der Kante steht dann besonders viel einkristallines Material zur Absorption zur Verfügung. Die Reinigung durch lonenstrahlätzen auch im zweiten Bereich verhindert, dass auf dessen Seite Rauigkeit oder Defekte die parasitäre Streuung unnötig erhöhen.

Vorteilhaft ist auch eine Verfahrensvariante, bei der in Schritt b) ein lonenstrahl eingesetzt wird, mit dem gleichzeitig mehrere einkristalline Blendenkörper bestrahlt werden. Die parallele Bearbeitung verringert bei größeren Stückzahlen die Zeit für Schritt b) pro Blende und kann so die Herstellungskosten senken.

In den Rahmen der vorliegenden Erfindung fällt weiterhin ein Verfahren zur Nachbearbeitung einer Röntgenblende, wobei die Röntgenblende einen Blendenkörper aufweist, der eine Kante zur Begrenzung eines Röntgenstrahls ausbildet, dadurch gekennzeichnet, dass Material von der Oberfläche des Blendenkörpers zumindest im Bereich der Kante durch lonenstrahlätzen abgetragen wird. Durch das lonenstrahlätzen werden die Defektdichte und die Rauigkeit im Bereich der Kante verringert sowie Verschmutzungen (und etwas Blendenmaterial) abgetragen. Dadurch kann die parasitäre Streuung verringert werden. Das Nachbearbeitungsverfahren wird typischerweise an einkristallinen Blendenkörpern angewandt; es ist aber auch eine Anwendung an polykristallinen Blendenkörpern im Rahmen der Erfindung möglich. Eine nachzubearbeitende Röntgenblende weist typischerweise nur einen Blendenkörper auf; die Erfindung kann aber auch mit Röntgenblenden umfassend mehrere Blendenkörper eingesetzt werden.

Erfindungsgemäß wurde die Röntgenblende vor der Materialabtragung durch das lonenstrahlätzen bereits in einem Röntgenanalysegerät eingesetzt. Dadurch kann eine erstmalige Verbesserung der Kantenqualität im Sinne einer Nachrüstung erreicht werden; es können aber auch im Laufe der Verwendung der Röntgenblende angesammelte Verunreinigungen und entstandene Defekte entfernt werden.

Eine bevorzugte Variante des Nachbearbeitungsverfahrens sieht vor, dass der Blendenkörper einkristallin ist und ein durchgehendes Loch aufweist, so dass die Kante am einkristallinen Blendenkörper als eine umlaufende, durchgehende Kante zur Begrenzung des Röntgenstrahls ausgebildet ist, dass von der Kante ausgehend sich das Loch in einem ersten Bereich in Richtung einer Austrittsöffnung trichterförmig erweitert, und dass Material von der Oberfläche des Blendenkörpers auch im ersten Bereich durch lonenstrahlätzen abgetragen wird, bevorzugt wobei das Loch zumindest im Bereich der Kante und des ersten Bereichs einen runden, insbesondere kreisrunden Querschnitt aufweist. Bei einem einkristallinen Blendenkörper kann die Kantenqualität durch das Ionenstrahlätzen besonders stark verbessert werden. Die runde Lochgeometrie gestattet die Verwendung eines runden Primärstrahlfängers bzw. ein verbessertes Auflösungsvermögen gegenüber quadratischen Strahlquerschnitten.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

Die Erfindung ist in der Zeichnung dargestellt und wird anhand von Ausführungsbeispielen näher erläutert. Es zeigen:
- Fig. 1a: eine schematische Übersicht über ein erfindungsgemäßes Röntgenanalysegerät, umfassend eine Röntgenblende mit einkristallinem Blendenkörper;
- Fig. 1b: eine schematische Übersicht über ein erfindungsgemäßes Röntgenanalysegerät, umfassend zwei Röntgenblenden mit einkristallinem Blendenkörper;
- Fig. 1c: eine schematische Übersicht über ein erfindungsgemäßes Röntgenanalysegerät, umfassend eine Blende mit einkristallinem Blendenkörper, mit beweglicher Röntgenquelle und beweglichem Röntgendetektor;
- Fig. 2: einen schematischen Querschnitt einer Röntgenblende mit einkristallinem Blendenkörper für die Erfindung, mit einem trichterförmigen, ersten Bereich;
- Fig. 3a: eine schematische Vorderansicht der Röntgenblende von Fig. 2, mit rundem Loch;
- Fig. 3b: eine schematische Rückansicht der Röntgenblende von Fig. 2, mit rundem Loch;
- Fig. 3c: ein Detailausschnitt von Fig. 2 im Bereich der Kante;
- Fig. 4a: eine schematische Vorderansicht einer Röntgenblende mit einkristallinem Blendenkörper, mit rechteckigem Loch;
- Fig. 4b: eine schematische Rückansicht der Röntgenblende von Fig. 4a;
- Fig. 5: einen schematischen Querschnitt einer Röntgenblende mit einkristallinem Blendenkörper für die Erfindung, mit einem trichterförmigen zweiten Bereich;
- Fig. 6a: eine schematische Querschnittsansicht einer Röntgenblende mit einkristallinem Blendenkörper für die Erfindung, mit einer Absorptionsstruktur auf der Vorderseite;
- Fig. 6b: eine schematische Querschnittsansicht einer Röntgenblende mit einkristallinem Blendenkörper für die Erfindung, mit einer Absorptionsstruktur auf der Rückseite;
- Fig. 6c: eine schematische Querschnittsansicht einer Röntgenblende mit einkristallinem Blendenkörper für die Erfindung, mit einer Absorptionsstruktur auf der Rückseite und einem hinteren Teil des trichterförmigen ersten Bereichs;
- Fig. 6d: eine schematische Querschnittsansicht einer Röntgenblende mit einkristallinem Blendenkörper für die Erfindung, mit einer Absorptionsstruktur auf der Vorderseite, der Rückseite und einem hinteren Teil des trichterförmigen ersten Bereichs;
- Fig. 7a: eine schematische Querschnittsansicht einer Röntgenblende mit einkristallinem Blendenkörper für die Erfindung, mit einer Absorptionsstruktur auf der Vorderseite, die eine über den Blendenkörper herausragende Halterung ausbildet;
- Fig. 7b: eine schematische Rückansicht der Röntgenblende von Fig. 7a;
- Fig. 7c: eine schematische Querschnittsansicht einer Röntgenblende mit einkristallinem Blendenkörper für die Erfindung, mit einer Absorptionsstruktur auf der Rückseite, die eine über den Blendenkörper herausragende Halterung ausbildet;
- Fig. 7d: eine schematische Querschnittsansicht einer Röntgenblende mit einkristallinem Blendenkörper für die Erfindung, mit einem einkristallinen Zusatzkörper und einer Absorptionsstruktur zwischen dem Blendenkörper und dem Zusatzkörper, wobei die Absorptionsstruktur eine über den Blendenkörper herausragende Halterung ausbildet;
- Fig. 8a: eine schematische Querschnittsansicht einer Röntgenblende mit einkristallinem Blendenkörper für die Erfindung, mit einer Abdeckstruktur auf der Rückseite;
- Fig. 8b: eine schematische Querschnittsansicht einer Röntgenblende mit einkristallinem Blendenkörper für die Erfindung, mit einer Abdeckstruktur auf der Vorderseite;
- Fig. 9: eine schematische Querschnittsansicht einer Röntgenblende mit einkristallinem Blendenkörper für die Erfindung, mit einer Füllstruktur im Loch des Blendenkörpers;
- Fig. 10a: eine schematische Darstellung einer Röntgenröhre mit strahlformender Optik und an dieser angeordneter Röntgenblende mit einkristallinem Blendenkörper für ein erfindungsgemäßes Röntgenanalysegerät;
- Fig. 10b: eine schematische Darstellung eines Kollimators, mit einer Röntgenblende mit einkristallinem Blendenkörper, für die Erfindung;
- Fig. 10c: eine schematische Darstellung eines Kollimators, mit zwei Röntgenblenden mit einkristallinem Blendenkörper, für die Erfindung;
- Fig. 11a: eine schematische Querschnittsdarstellung eines einkristallinen Blendenkörpers gehalten in klemmenartigen seitlichen Führungen während einer erfindungsgemäßen Nachbehandlung durch lonenätzen von der Rückseite;

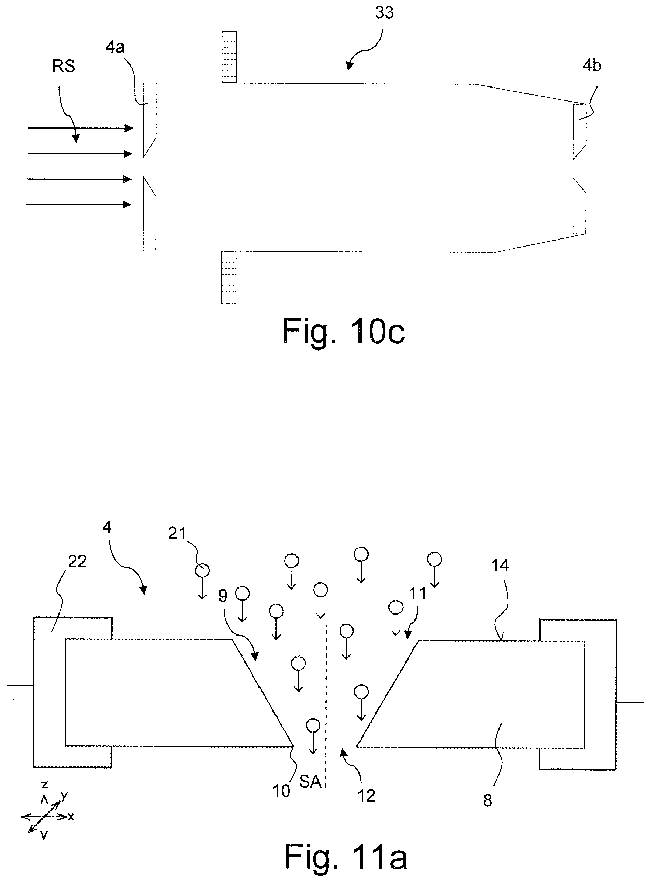

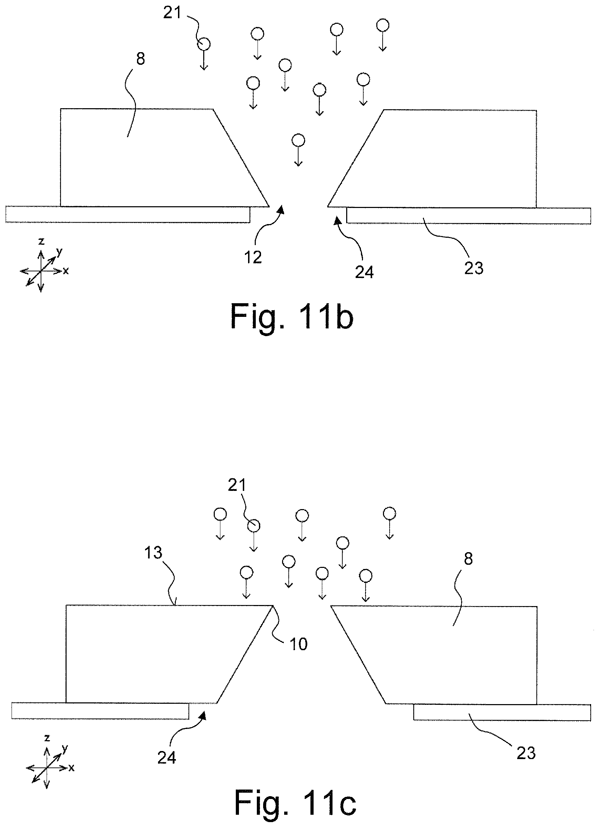

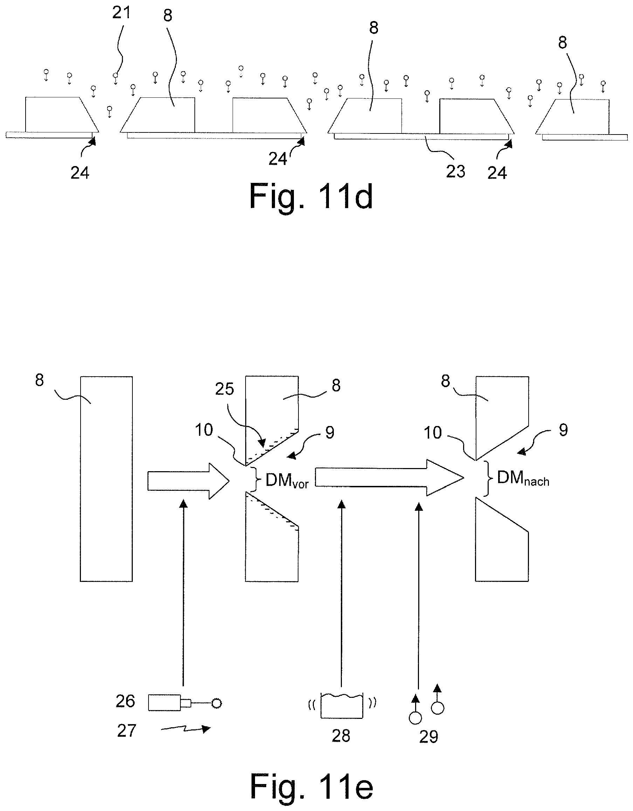

- Fig. 11 b: eine schematische Querschnittsdarstellung eines einkristallinen Blendenkörpers aufliegend auf einer gelochten Auflageplatte während einer erfindungsgemäßen Nachbehandlung durch lonenätzen von der Rückseite;
- Fig. 11c: eine schematische Querschnittsdarstellung eines einkristallinen Blendenkörpers aufliegend auf einer gelochten Auflageplatte während einer erfindungsgemäßen Nachbehandlung durch Ionenätzen von der Vorderseite;
- Fig. 11d: eine schematische Querschnittsdarstellung mehrerer einkristalliner Blendenkörper, aufliegend auf einer gemeinsamen, mehrfach gelochten Auflageplatte, während einer gleichzeitigen erfindungsgemäßen Nachbehandlung durch lonenätzen von der Rückseite;
- Fig. 11e: eine schematische Übersicht über den erfindungsgemäßen Herstellungsprozess einer Röntgenblende mit einkristallinem Blendenkörper;

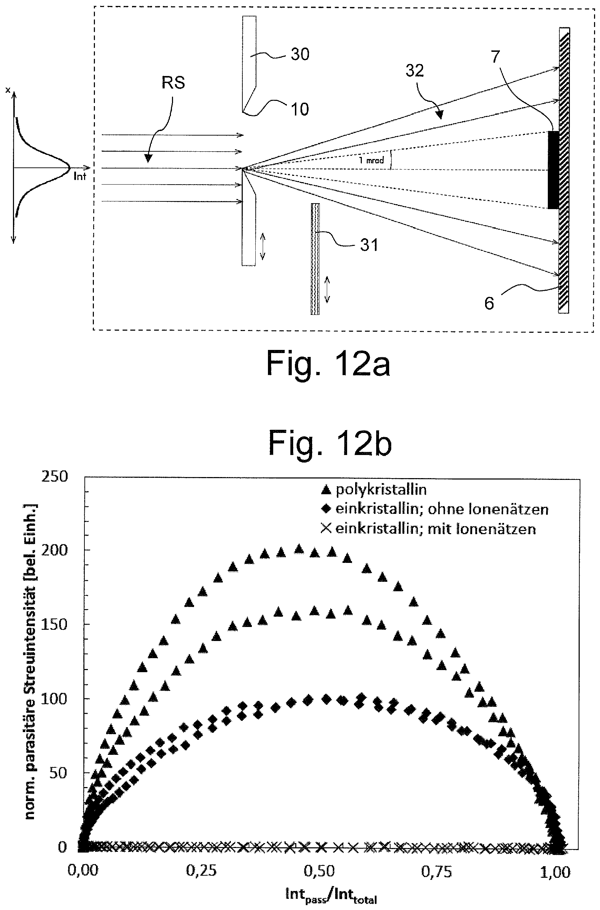

- Fig. 12a: eine schematische Übersicht über einen Messaufbau zur Bestimmung der parasitären Streuung einer Röntgenblende;
- Fig. 12b: ein Diagramm darstellend die parasitären Streuintensitäten einer polykristallinen (▲), einer einkristallinen nicht ionengeätzen (◆) sowie einer erfindungsgemäß einkristallinen ionengeätzten Röntgenblende (x) bei verschiedenem Überlapp des Röntgenstrahls mit der Röntgenblende;

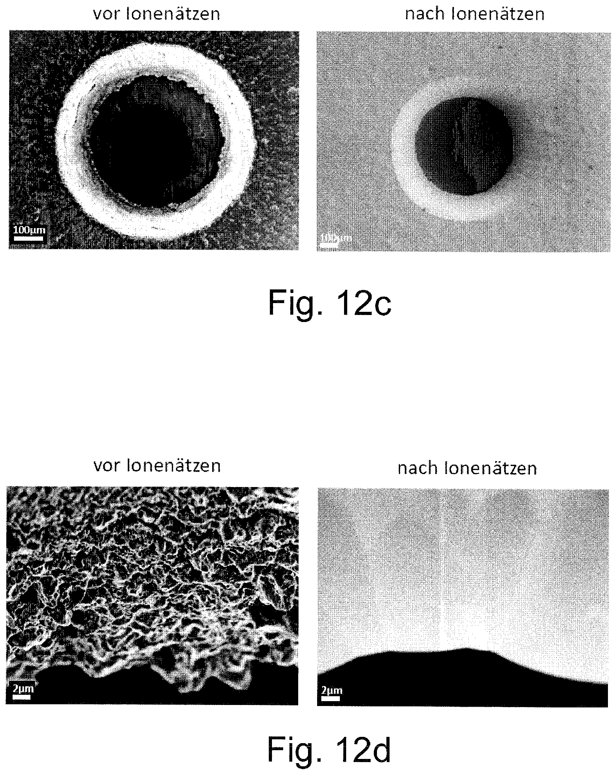

- Fig. 12c: REM-Bilder der Rückseite einer einkristallinen Röntgenblende für die Erfindung, vordem lonenätzen (links) und nach dem lonenätzen (rechts);
- Fig. 12d: REM-Bilder der Kante einer einkristallinen Röntgenblende für die Erfindung, vor dem lonenätzen (links) und nach dem lonenätzen (rechts).

### Überblick über die Erfindung

Mit der vorliegenden Erfindung wird eine kompakte, streufreie (oder zumindest streuarme), bevorzugt runde Apertur für ein Röntgenanalysegerät vorgestellt; die Apertur wird auch als Röntgenblende bezeichnet. Die Apertur hat den Vorteil, dass sie leicht und reproduzierbar herstellbar ist sowie leicht und kostengünstig in bestehende und neue Röntgenanalysegeräte, speziell für SAXS-, SCD- und µ-XRD-Untersuchungen, integriert und justiert werden kann. Bekannte Herstellungsverfahren für Röntgenblenden können entweder das geforderte Aperturdesign nicht herstellen oder aber bringen Streuzentren in die Apertur ein. Die Erfindung stellt daher auch ein Herstellungsverfahren bereit, mit denen Aperturen für die Erfindung bereitgestellt werden können.

Hierbei wird ein spezielles Nachbearbeitungsverfahren (umfassend eine Oberflächenbearbeitung durch lonenstrahlätzen, vgl. Schritt b) von Anspruch 13) angewandt, das sich an das Einbringen eines Loches in einen einkristallinen Blendenkörper anschließt. Das Nachbearbeitungsverfahren wird dazu eingesetzt, bereits hergestellte Aperturen, die bereits in einem Röntgenanalysegerät eingesetzt wurden, so zu modifizieren, dass sie streufrei (oder streuärmer) werden.

Gemäß der Erfindung wird in einen Einkristall ein sich in Strahlrichtung aufweitender Tunnel mit vorzugsweise runder Querschnittsfläche eingebracht, wobei der Rand der minimalen und damit strahldefinierenden Querschnittsfläche des Tunnels eine möglichst scharfe Kante ausbildet.

Die Verwendung eines einzelnen, kompakten Einkristalls ermöglicht es, parasitäre Streuung an Körnern, Korngrenzen und weiteren Defekten zu vermeiden. Weiterhin wird die durch Totalreflexion verursachte parasitäre Streuung dadurch verhindert bzw. reduziert, dass der sich in Strahlrichtung aufweitende Tunnel einen Öffnungswinkel größer als der Totalreflexionswinkel hat und die Kante am Rand der strahldefinierenden Querschnittsfläche möglichst scharf, bestenfalls atomar scharf ist.

Die äußeren Abmessungen des Einkristalls können den kommerziell erhältlichen, parasitär streuenden, polykristallinen Aperturen, welche in gängigen Röntgenanalysegeräten verbaut werden, entsprechen, wodurch ein Aperturwechsel schnell, einfach und kostengünstig realisiert werden kann. Ein solcher Aperturwechsel zu einer Röntgenblende, mit der ein erfindungsgemäßes Röntgenanalysegerät eingerichtet wird, kann auch deshalb schnell erfolgen, weil verglichen mit herkömmlichen, streuenden Blenden die streufreien (oder zumindest streuarmen) Aperturen mit einkristallinem Blendenkörper schneller entlang des Strahlpfades ausgerichtet werden können, da sie weniger genau justiert werden müssen: Eine geringfügige Fehljustierung vergrößert nicht oder nur wenig die Hintergrundstrahlung, so dass das Signal-Rausch-Verhältnis nicht wesentlich verschlechtert wird.

Die streufreien (oder zumindest streuarmen) Aperturen mit einkristallinem Blendenkörper können in einigen wenigen Kombinationen von an sich bekannten Bearbeitungsverfahren hergestellt werden; ein maschinelles, reproduzierbares und standardisiertes Einbringen eines sich in Strahlrichtung aufweitenden Tunnels ist mit diesen Kombinationen gut möglich.

Die erfindungsgemäßen Bearbeitungsverfahren ermöglichen es, neben anderen Formen auch eine runde Querschnittsfläche zu wählen, so dass die Auflösung von SAXS-Analysegeräten, verglichen mit streufreien Hybridschneidensystemen, um über 40% verbessert werden kann.

Im Rahmen der Erfindung werden zum Einbringen des Lochs (Schritt a) des Anspruchs 13) nur solche Bearbeitungsverfahren eingesetzt, welche Defekte im Wesentlichen nahe der Oberfläche des Einkristalls, jedoch nicht oder nur in geringem Ausmaß in größerer Tiefe einbringen, insbesondere Funkenerosion oder kalte Laserablation. Andere Verfahren wie z.B. Bohren sind deswegen ungeeignet. Die eingebrachten Defekte, sowie mögliche Verschmutzungen und große Oberflächenrauigkeit verursachen relativ starkes, parasitäres Streuen. Daher werden erfindungsgemäß in einem Nachbearbeitungsschritt (Schritt b) des Anspruchs 13) stark beschleunigte Ionen auf die Einkristalloberfläche gelenkt, so dass die Ionen die defektreichen Oberflächenschichten sowie Absorbate und Adsorbate abtragen und die Oberflächenrauigkeit reduzieren. Durch die damit verbundene Verringerung der Anzahl potentieller Streuzentren wird auch das parasitäre Streuverhalten der Apertur stark verbessert, d.h. die Streuung reduziert.

Einkristallbasierte (d.h. auf einem einkristallinem Blendenkörper basierende), streufreie (oder zumindest streuarme) Aperturen ermöglichen es, die Anzahl der Lochblenden in einem SAXS-Aufbau auf eins oder zwei zu reduzieren, wodurch höhere Photonenflüsse und kleinere Primärstrahlfänger möglich sind, was wiederum die Auflösung erhöht und die Messzeiten verkleinert. Außerdem wird der Platzbedarf eines SAXS-Messinstruments stark reduziert.

Die erfindungsgemäßen streufreien Blenden ermöglichen es in einem SCD-Aufbau, kleinere Primärstrahlfänger zu verwenden, was zu einer höheren Auflösung um den Primärstrahl führt.

Die auf einen einkristallinen Blendenkörper basierenden, streufreien (oder zumindest streuarmen) Lochblenden ermöglichen bei einem µ-XRD-Messaufbau größere Lochblendendurchmesser, wodurch sich höhere Photonenflüsse und damit kürzere Messzeiten ergeben. Außerdem erzeugen die einkristallinen Aperturen keine das Messsignal überlagernden Debye-Scherrer-Ringe auf dem Detektor, sondern einzelne Beugungsreflexe, welche dem Blendenmaterial eindeutig zugeordnet werden können und damit die Auswertung der Messdaten erleichtern.

### Erfindungsgemäße Röntgenanalysegeräte

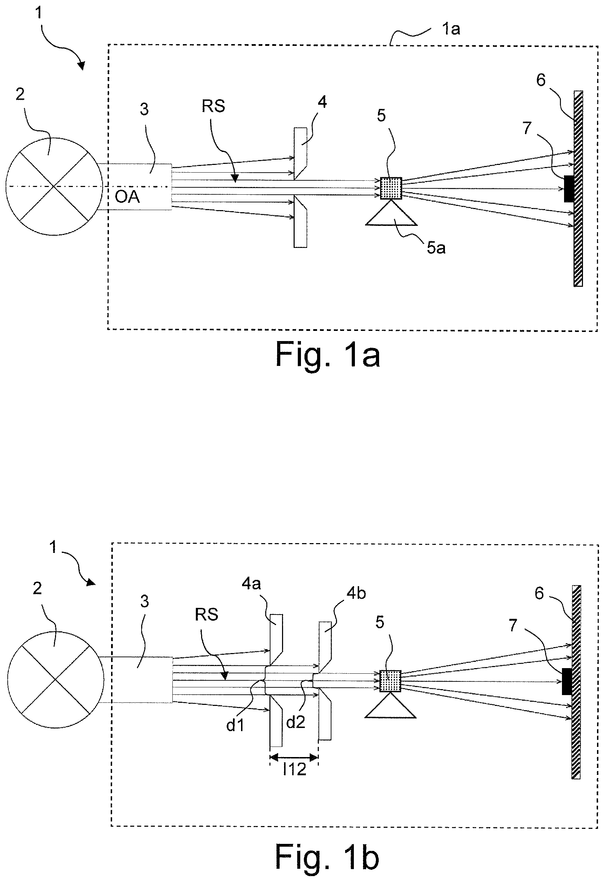

Die **Figur 1a** zeigt eine erste Ausführungsform eines erfindungsgemäßen Röntgenanalysegeräts 1 in einer Schemadarstellung. Das Röntgenanalysegerät 1 kann beispielsweise für eine SAXS- oder SCD-Messung eingesetzt werden.

Das Röntgenanalysegerät 1 umfasst eine Röntgenquelle 2, beispielsweise eine Röntgenröhre, am einfachsten mit fester Anode. Ein von der Röntgenquelle 1 emittierter Röntgenstrahl RS wird von einem strahlformenden Element 3 umgeformt (hier von einem Göbel- bzw. Montelspiegel näherungsweise parallelisiert) und auf eine Röntgenblende 4 gerichtet. In der gezeigten Ausführungsform ist das strahlformende Element 3 direkt an der Röntgenquelle 2 befestigt und die Röntgenblende 4 ist vom strahlformenden Element 3 beabstandet.

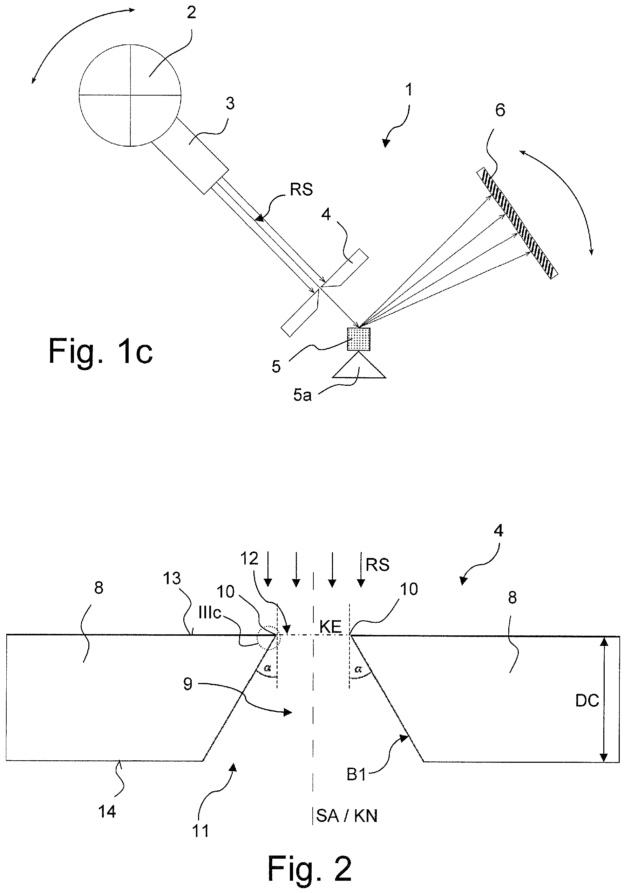

Die Röntgenblende 4 weist einen einkristallinen Blendenkörper auf (siehe dazu unten, beispielsweise Fig. 2) und weitet sich in Ausbreitungsrichtung des Röntgenstrahls RS (in Fig. 1 von links nach rechts, vgl. optische Achse OA) trichterförmig auf. Die Röntgenblende 4 blockt einen äußeren Teil des Röntgenstrahls RS ab, wobei keine oder nur sehr wenig parasitäre Streustrahlung erzeugt wird. Dadurch ist es möglich, mit nur einer Röntgenblende 4 auszukommen, und gleichzeitig eine geringe Strahldivergenz einzuhalten. Im Falle einer SCD-Messung macht die Röntgenblende 4 es möglich, einen besonders kleinen Primärstrahlfänger (siehe unten) zu verwenden, wodurch eine erhöhte Auflösung erreicht wird.

Der Röntgenstrahl RS trifft sodann auf eine Probe 5, welche auf einem Probenhalter bzw. Goniometer 5a gehalten ist und welche von der Röntgenblende 4 beabstandet ist, typischerweise wenigstens 1cm weit in Strahlausbreitungsrichtung bei SAXS- und SCD- Messungen. Der Röntgenstrahl RS wechselwirkt mit der Probe 5, was in einer von der Probe 5 ausgehenden Röntgenstrahlung resultiert, die von einem Röntgendetektor 6 registriert wird. Der Röntgendetektor 6 ist hier als Flächendetektor ausgebildet und ist wiederum von der Probe 5 beabstandet, typischerweise in einem Bereich von 10cm bis 100cm bei SAXS- Messungen und typischerweise wenigstens 3cm bei SCD-Experimenten in Strahlausbreitungsrichtung. Der Detektor 6 wird dabei durch einen Primärstrahlfänger 7 in einem zentralen Bereich abgeschattet, so dass transmittierte ungebeugte bzw. ungestreute Röntgenstrahlung nicht auf den Detektor 6 trifft.

In der gezeigten Ausführungsform ist der Strahlengang der Röntgenstrahlung im Wesentlichen in einem evakuierten Gehäuse 1a angeordnet (wobei Teile des Strahlengangs auch unter Schutzgasatmosphäre gehalten sein können), so dass Luftstreuung minimiert ist.

Die **Figur** 1**b** zeigt eine zweite Ausführungsform eines erfindungsgemäßen Röntgenanalysegeräts 1, welches weitgehend der Ausführungsform von Fig. 1a entspricht. Im Folgenden sollen daher nur die wesentlichen Unterschiede aufgezeigt werden.

Das Röntgenanalysegerät 1 verfügt über zwei Röntgenblenden 4a, 4b mit einkristallinem Blendenkörper (siehe dazu unten) auf, die sich jeweils in Strahlausbreitungsrichtung trichterförmig aufweiten. Die Röntgenblenden 4a, 4b blocken jeweils einen äußeren Teil des Röntgenstrahls RS ab, wobei keine oder nur sehr wenig parasitäre Streustrahlung erzeugt wird.

Die Röntgenblenden 4a, 4b sind zwischen Röntgenquelle 2 und Probe 5 angeordnet. Beide Röntgenblenden 4a, 4b sind von der Probe 5 beabstandet angeordnet. Bei SAXS-Messanordnungen werden dabei die Blendendurchmesser d1, d2 und der Blendenabstand 112 entsprechend der benötigten Divergenz und dem Strahlenquerschnitt des jeweiligen Messaufbaus gewählt (Man beachte, dass die Blendendurchmesser d1, d2 der Röntgenblenden 4a, 4b gleich oder - wie hier dargestellt - unterschiedlich gewählt werden können). Blendendurchmesser d1, d2 und Blendenabstand 112 sollten an den Fokus bzw. die Divergenz der Röntgenquelle 2 angepasst sein, um die Leistung der Röntgenquelle 2 bestmöglich auszunutzen. Dabei ist typischerweise die Röntgenblende 4a wenigstens 20 cm und die Röntgenblende 4b wenigstens 1 cm weit in Strahlausbreitungsrichtung von der Probe 5 beabstandet. Bei SCD-Experimenten sind die Röntgenblenden 4a, 4b von der Probe 5 typischerweise wenigstens 8cm (Röntgenblende 4a) und wenigstens 1 cm (Röntgenblende 4b) weit in Strahlausbreitungsrichtung von der Probe 5 beabstandet.

Die **Figur 1c** zeigt schematisch in Aufsicht eine dritte Ausführungsform eines erfindungsgemäßen Röntgenanalysegeräts 1, welches insbesondere als µ-XRD-Messanordnung geeignet ist. Wiederum werden nur die wesentlichen Unterschiede zur Ausführungsform von Fig. 1a erläutert.

Die Röntgenquelle 2 ist hier, zusammen mit dem strahlformenden Element 3 und der Röntgenblende 4 mit einkristallinem Blendenkörper, relativ zur Probe 5 auf dem Probenhalter 5a drehbar, wobei die zugehörige Drehachse senkrecht zur Zeichenebene von Fig. 1c verläuft; um die gleiche Drehachse ist auch der Detektor 6 drehbar (Goniometer-Aufbau). Durch die Verwendung der Röntgenblende 4 mit einkristallinem Blendenkörper kann mit großen Blendendurchmessern bzw. hohen Photonenflüssen gearbeitet werden und gleichzeitig der Röntgenstrahl RS scharf auf die Probe 5 oder aber auf eine kleine Fläche bzw. ein kleines Volumen der Probe 5 begrenzt werden.

**Figur 2** zeigt in einer Querschnittsdarstellung eine Röntgenblende 4 (wie sie in einem Röntgenanalysegerät 1 aus Fig. 1a oder Fig. 1b verbaut sein kann) basierend auf einem einkristallinen Blendenkörper gemäß der Erfindung.

Die Röntgenblende 4 umfasst einen einzigen, einkristallinen (monokristallinen) Blendenkörper 8 mit einem durchgehenden Loch 9. Das Loch 9 ist an einer umlaufenden, ununterbrochenen Kante 10 am engsten. Diese Kante 10 begrenzt einen Röntgenstrahl RS, welcher in Fig. 2 von oben die Röntgenblende 4 trifft; der Röntgenstrahl RS breitet sich dabei näherungsweise parallel zu einer Symmetrieachse SA der Röntgenlende 4 aus. Die umlaufende Kante 10 verläuft hier in einer Ebene (vgl. Kantenebene KE, strichpunktiert eingezeichnet) senkrecht zur Symmetrieachse SA (d.h. die Kantenebenen-Flächennormale KN verläuft parallel zur Symmetrieachse SA); die Kante 10 bestimmt die "strahldefinierende Querschnittsfläche" des Lochs 9.

An die Kante 10 schließt sich in Ausbreitungsrichtung des Röntgenstrahls RS ein erster Bereich B1 an, der sich trichterförmig in Richtung einer Austrittsöffnung 11 der Röntgenblende 4 aufweitet. Der erste Bereich B1 ist hier kegelstumpfartig ausgebildet, mit einem Öffnungswinkel α von ca. 30° zwischen der Mantelfläche des ersten Bereichs B1 und der Symmetrieachse SA (in Fig. 2 sind zusätzlich zur Symmetrieachse SA parallelverschobene Hilfslinien eingezeichnet, um den Öffnungswinkel α besser kenntlich zu machen).

In der gezeigten Ausführungsform definiert die Kante 10 gleichzeitig eine Eintrittsöffnung 12 der Röntgenblende 4; die Vorderseite (Auftreffseite) 13 des Blendenkörpers 8 verläuft bis zur Kante 10 senkrecht zur Symmetrieachse SA. Mit anderen Worten ist die Kante 10 an der Vorderseite 13 ausgebildet. Die Blendendicke DC, das ist der Abstand zwischen Vorderseite 13 und Rückseite (Austrittsseite) 14 der Röntgenblende 4 bzw. des Blendenkörpers 8, liegt typischerweise in einem Bereich von 0,1 mm bis 3 mm, bevorzugt von 0,5 mm bis 1,5 mm. Der Blendenkörper 8 wird typischerweise aus Si oder Ge hergestellt (etwa einem einkristallinen Wafer).

Vorderseite 13 und Rückseite 14 des Blendenkörpers 8 der Röntgenblende von Figur 2 sind auch in **Figur 3a** (Vorderansicht) und **Figur 3b** (Rückansicht), die die Röntgenblende 4 jeweils in einer Aufsicht in einer Richtung entlang Symmetrieachse SA (die senkrecht zu den Bildebenen verläuft) zeigen. Das Loch 9 weist sowohl im Bereich der Kante 10 als auch im ersten Bereich B1 einen kreisrunden Querschnitt auf; dadurch kann ein Primärstrahlfänger mit minimaler (kreisrunder) Abschattungsfläche eingesetzt werden. Die Kante 10 ist über den gesamten Umlauf ununterbrochen; der einkristalline Blendenkörper 8 ist einstückig ausgebildet. In den Figuren 3a und 3b ist die strahldefinierende Querschnittsfläche des Lochs 9 jeweils unmittelbar ersichtlich.

Die **Figur 3c** zeigt einen vergrößerten Ausschnitt der in Fig. 2 dargestellten Röntgenblende (vgl. dortige gestrichelte Kreismarkierung IIIc) im Bereich der Kante 10. Im Querschnitt mit der Querschnittsfläche parallel zur Ausbreitungsrichtung des Röntgenstrahls RS bzw. parallel zur Symmetrieachse ergibt sich in der Praxis typischerweise ein abgerundetes Profil 37 des einkristallinen Blendenkörpers 8, wobei näherungsweise gerade Profilabschnitte (hier der Vorderseite 13 und des ersten trichterförmigen Bereichs B1) auf die Kante 10 zulaufen und im Bereich der Kante 10 gebogen ineinander übergehen.

Vorteilhafter Weise ist die Kante 10 dabei möglichst scharf ausgebildet, bevorzugt mit einem Kantenradius kleiner 5 µm. Der Kantenradius entspricht hierbei dem Radius R des größtmöglichen Kreises 35, der von innen (also vom Blendenkörper 8 aus) an das Profil 37 angelegt werden kann, ohne das Profil 37 zu schneiden. Der Mittelpunkt 36 des Kreises 35 liegt in der gezeigten Ausführungsform auf einer Winkelhalbierenden 38 zwischen den geraden Profilabschnitten der Vorderseite 13 und des ersten Bereichs B1; der Kreis 35 liegt zumindest am Schnittpunkt der Winkelhalbierenden 38 mit dem Profil 37 am Profil 37 an. Durch das erfindungsgemäße Nachbearbeitungsverfahren mit lonenätzen ist die Ausbildung einer Kante 10 mit einem Kantenradius kleiner 5 µm über den gesamten Lochumfang problemlos möglich.

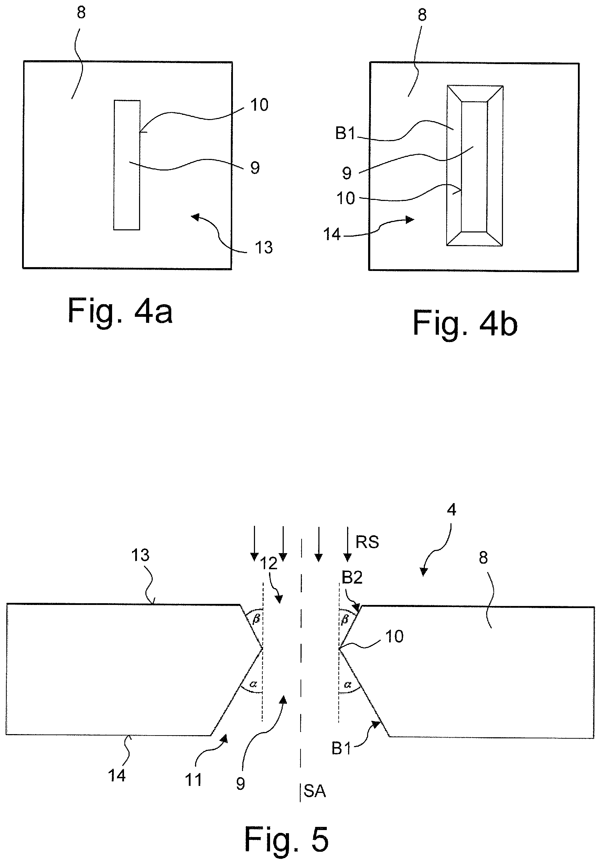

In einer alternativen Ausführungsform einer Röntgenblende 4 mit einkristallinem Blendenkörper 8 weist die Röntgenblende 4 ein schlitzförmiges Loch 9 auf, wie in **Figur 4a** in Vorderansicht und **Figur 4b** in Rückansicht ersichtlich. Der erste Bereich B1 verläuft auch in diesem Falle trichterförmig, hier jedoch mit vier ebenen, aber gegenüber der Symmetrieachse (die senkrecht zu den Bildebenen verläuft) schrägstehenden Lochwandabschnitten. Auch hier ist die umlaufende Kante 10 ununterbrochen, und der einkristalline Blendenkörper 8 ist einstückig. Man beachte, dass bei der Herstellung des im Querschnitt rechteckigen Lochs 9 bei geeigneter Ausrichtung des Blendenkörpers 8 und geeignetem Blendenkörpermaterial Anisotropien in der Materialabtragungsrate bezüglich verschiedener Richtungen des Kristallgitters des einkristallinen Blendenkörpers 8 genutzt werden können. In den Figuren 4a und 4b ist wiederum die strahldefinierende Querschnittsfläche des Lochs 9 jeweils unmittelbar ersichtlich.

In **Figur 5** ist eine weitere Ausführungsform einer Röntgenblende 4 basierend auf einem einkristallinem Blendenkörper 8 im Querschnitt dargestellt, bei der sich auch in einem zweiten Bereich B2, der sich von der Kante 10 weg in Richtung der Eintrittsöffnung 12 erstreckt, das Loch 9 in besagter Richtung trichterförmig erweitert. Im zweiten Bereich B2 hat das Loch 9 hier einen kegelstumpfartigen Verlauf (mit kreisrundem Querschnitt, nicht sichtbar in Fig. 5), mit einem Öffnungswinkel β von ca. 25°, gemessen zwischen der Mantelfläche im zweiten Bereich B2 und der Symmetrieachse SA der Röntgenblende 4 (der Winkel β ist in der Figur an einer zur Symmetrieachse SA parallelverschobenen Hilfslinie eingezeichnet).

Im ersten Bereich B1 verläuft das Loch 9 wie in Fig. 2 dargestellt.

Durch die beiden trichterförmigen Bereiche B1 und B2 , die sich an die Kante 10 anschließen, ist im Bereich der Kante 10 mehr absorbierendes Material als in der Ausführungsform von Fig. 2 angeordnet. Dadurch kann eine schärfere Begrenzung des Röntgenstrahls RS erreicht werden.

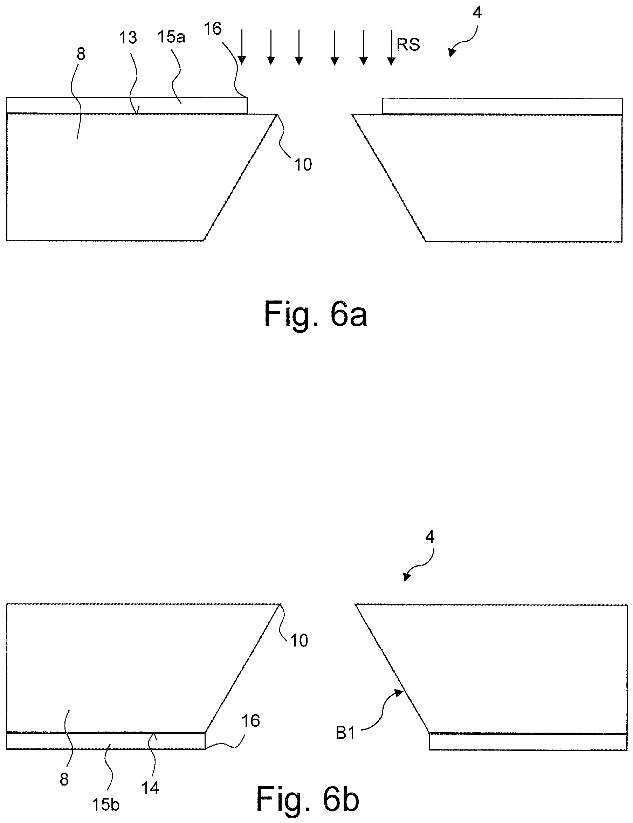

Die Figuren 6a bis 6d zeigen jeweils Röntgenblenden 4 basierend auf einem einkristallinen Blendenkörper 8, ähnlich wie in Figur 2 dargestellt, jedoch jeweils mit einer Absorptionsstruktur 15a-15d versehen. Die Absorptionsstruktur 15a-15d ist typischerweise mit dem Blendenkörper 8 verklebt; die Absorptionsstruktur 15a-15d ist beispielsweise aus polykristallinem Blei gefertigt.

Die Absorptionsstruktur 15a in **Figur 6a** ist an der Vorderseite 13 der Röntgenblende 4 angeordnet. Sie ist über den gesamten Umfang gegenüber der Kante 10 quer zur Strahlausbreitungsrichtung des Röntgenstrahls RS zurückgesetzt, vgl. die innere Kante 16 der Absorptionsstruktur 15a: Auf der linken Seite der Röntgenblende 4 im Querschnitt der Figur 6a ist die innere Kante 16 weiter links liegend als die innere Kante 10; entsprechendes gilt auch für die rechte Seite der Röntgenblende 4. Dadurch ist sichergestellt, dass an der Absorptionsstruktur 15a, insbesondere an der inneren Kante 16 gestreute Röntgenstrahlung vom Blendenkörper 8 abgeblockt werden kann.

Die Absorptionsstruktur 15b in **Figur 6b** ist an der Rückseite 14 der Röntgenblende 4 angeordnet. Obwohl die Absorptionsstruktur 15b hier die gesamte Rückseite 14 überdeckt, ist aufgrund des sich aufweitenden ersten Bereichs B1 auch hier sichergestellt, dass die innere Kante 16 gegenüber der Kante 10 des Blendenkörpers 8 zurückgesetzt ist.

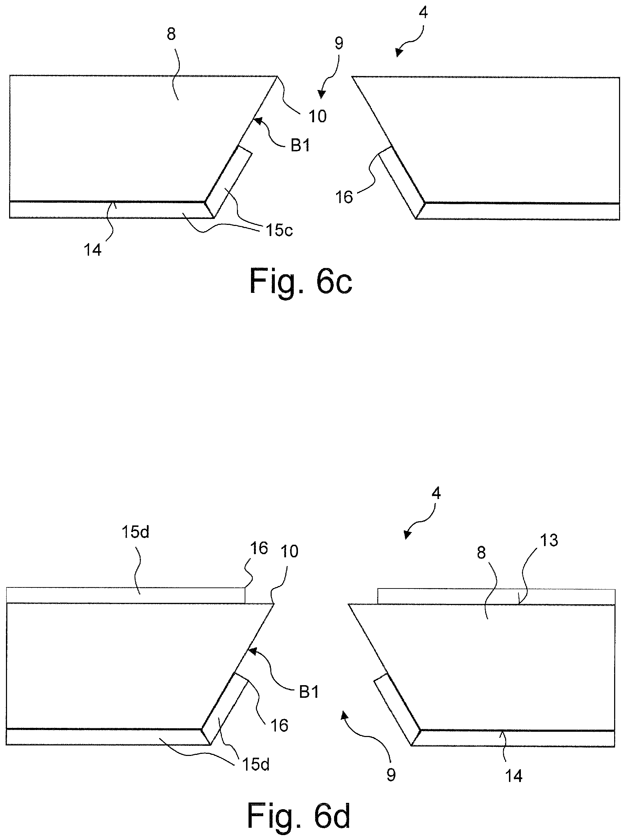

In der Absorptionsstruktur 15c von **Figur 6c** ist neben der Rückseite 14 auch ein Teil des trichterförmigen, ersten Bereichs B1 des Lochs 9 ausgekleidet. Dadurch rückt die innere Kante 16 radial etwas näher an die Kante 10 des Blendenkörpers 8; aber auch hier ist die innere Kante 16 gegenüber der Kante 10 zurückgesetzt.

Die Absorptionsstruktur 15d von **Figur 6d** überdeckt schließlich den größten Teil der Vorderseite 13, einen hinteren Teil des ersten Bereichs B1 und die Rückseite 14 des Blendenkörpers 8. Dadurch wird ein Maximum an Absorption von Röntgenstrahlung, die nicht durch das Loch 9 gelangt, sichergestellt. Beide inneren Kanten 16 der Absorptionsstruktur 15d sind gegenüber der Kante 10 zurückgesetzt.

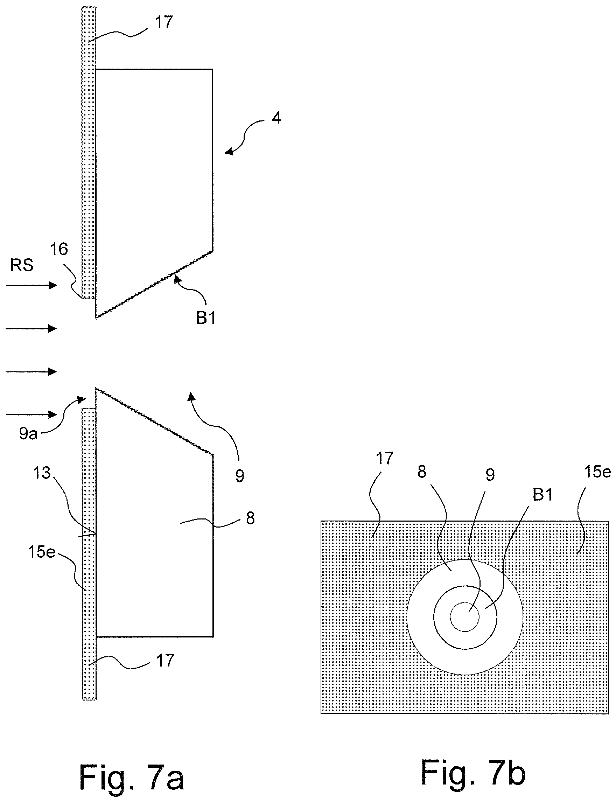

Die **Figur 7a** illustriert eine weitere Ausführungsform einer Röntgenblende 4 basierend auf einem einkristallinen Blendenkörper 8, welche an der Vorderseite 13 eine Absorptionsstruktur 15e ähnlich wie in Fig. 6a dargestellt aufweist, wobei jedoch die Absorptionsstruktur 15e quer zur Ausbreitungsrichtung des Röntgenstrahls RS außen über den Blendenkörper 8 hinausragt und dadurch eine Halterung 17 ausbildet. Die Halterung 17 kann beispielsweise von Klemmen (nicht dargestellt) erfasst werden, um die Röntgenblende 4 zu halten, ohne dass der meist fragile Einkristall des Blendenkörpers 8 (der beispielsweise aus Silizium oder Germanium gefertigt ist) mechanisch belastet wird. Man beachte, dass in der Absorptionsstruktur 15e im Bereich des kreisrunden Lochs 9 des Blendenkörpers 8 ein hier ebenfalls kreisrundes Loch 9a ausgebildet ist, welches die innere Kante 16 der Absorptionsstruktur 15e definiert. Die **Figur 7b** zeigt die Röntgenblende 4 von Fig. 7a in einer rückwärtigen Aufsicht (senkrecht zur Strahlausbreitungsrichtung).

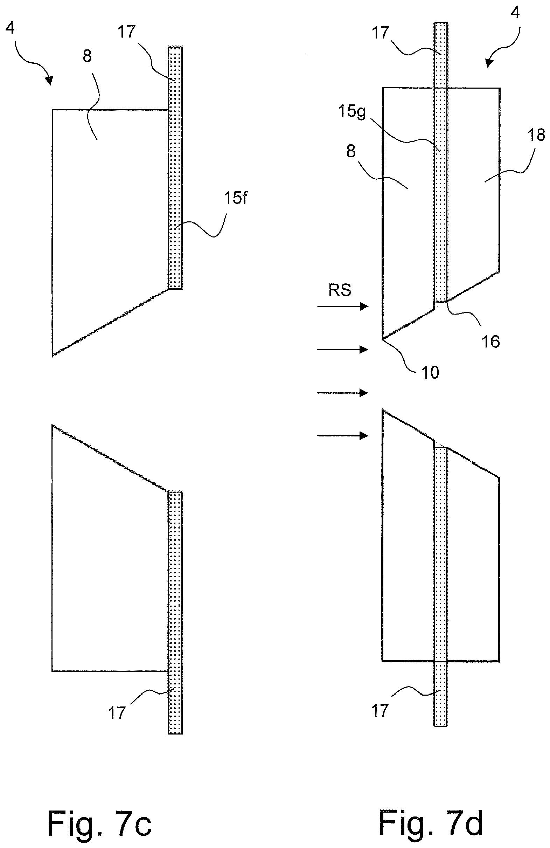

**Figur 7c** zeigt eine Röntgenblende 4 ähnlich Fig. 6b mit rückseitiger Absorptionsstruktur 15f, wobei wiederum die Absorptionsstruktur 15f über den Blendenkörper 8 hinausragt, um eine Halterung 17 auszubilden.

In **Figur 7d** wird eine Röntgenblende 4 mit einkristallinem Blendenkörper 8 für die Erfindung dargestellt, bei der ein einkristalliner Zusatzkörper 18 eingesetzt wird. Der Zusatzkörper 18 und der Blendenkörper 8 sind an gegenüberliegenden Seiten der Absorptionsstruktur 15g befestigt, typischerweise angeklebt. Die Kante 10, welche den Röntgenstrahl RS begrenzt, ist am Blendenkörper 8 ausgebildet. Die innere Kante 16 der Absorptionsstruktur 15g ist gegenüber der Kante 10 zurückgesetzt. Die Absorptionsstruktur 15g bildet wiederum Halterungen 17 aus, die seitlich über den Blendenkörper 8 herausragen. Der Zusatzkörper 18 wird meist mit einem anderen Material als der Blendenkörper 8 gewählt, um ggf. Schwächen des Blendenkörpermaterials - etwa bei verschiedenen Anwendungen unter Einsatz von Röntgenstrahlung unterschiedlicher Wellenlänge - ausgleichen zu können.

In allen Ausführungsformen können die Halterungen 17 Rillen, Vorsprünge, Vertiefungen, Gewinde oder dergleichen mehr ausbilden, um eine Befestigung der Röntgenblende 4 zu erleichtern.

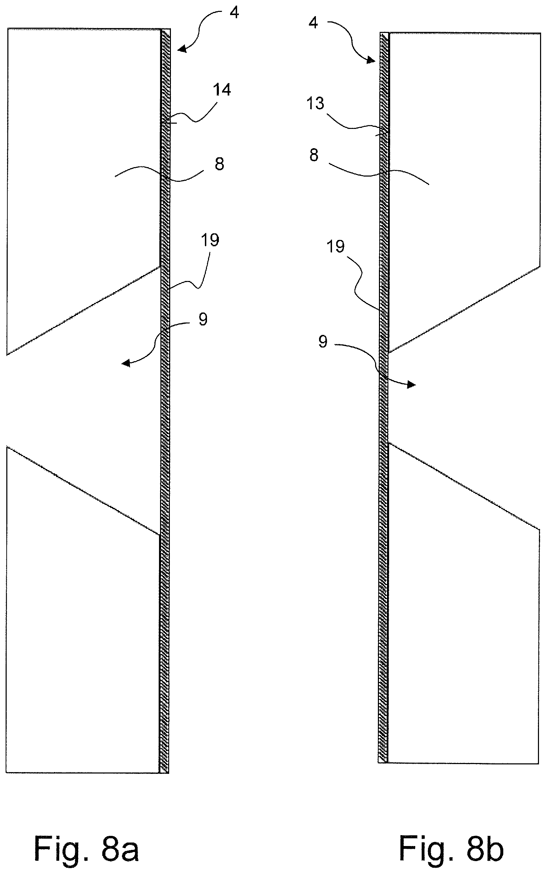

Die **Figur 8a** zeigt eine Röntgenblende 4 mit einkristallinem Blendenkörper 8 für die Erfindung, die eine Abdeckstruktur 19 aufweist; diese ist in der gezeigten Ausführungsform an der Rückseite 14 des Blendenkörpers 8 angeordnet und überdeckt das Loch 9 vollständig, so dass dieses gasdicht und vakuumdicht verschlossen wird. Typischerweise besteht die Abdeckstruktur 19 aus Beryllium oder Kapton. Die Röntgenblende 4 kann durch die Abdeckstruktur 19 als Röntgenfenster dienen und Teile des Strahlenganges mit unterschiedlichem Gasdruck voneinander trennen. Die Abdeckstruktur 19 ist typischerweise auf den Blendenkörper 8 aufgeklebt.

Wie aus **Figur 8b** ersichtlich, kann die Abdeckstruktur 19 alternativ auch an der Vorderseite 13 des Blendenkörpers 8 angeordnet sein, um das Loch 9 abzudichten.

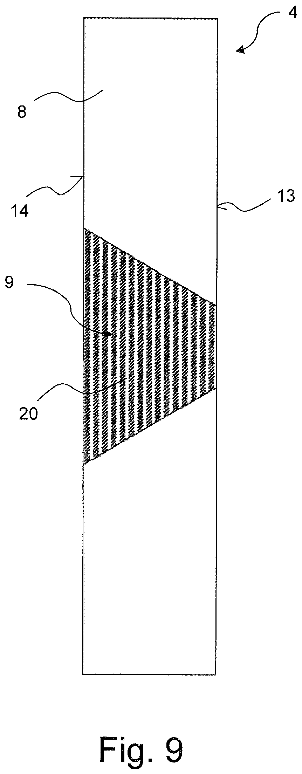

Die **Figur 9** zeigt weiterhin eine Röntgenblende 4 mit einkristallinem Blendenkörper 8 für die Erfindung, bei der eine Füllstruktur 20 das durchgehende Loch 9 ausfüllt. Die Füllstruktur 20 besteht aus einem (im Vergleich zum Material des Blendenkörpers 8) für Röntgenstrahlung schwach absorbierenden Material, etwa Beryllium oder Kapton. Die Füllstruktur 20 ist in der gezeigten Ausführungsform bündig sowohl mit der Vorderseite 13 als auch mit der Rückseite 14 des Blendenkörpers 8.

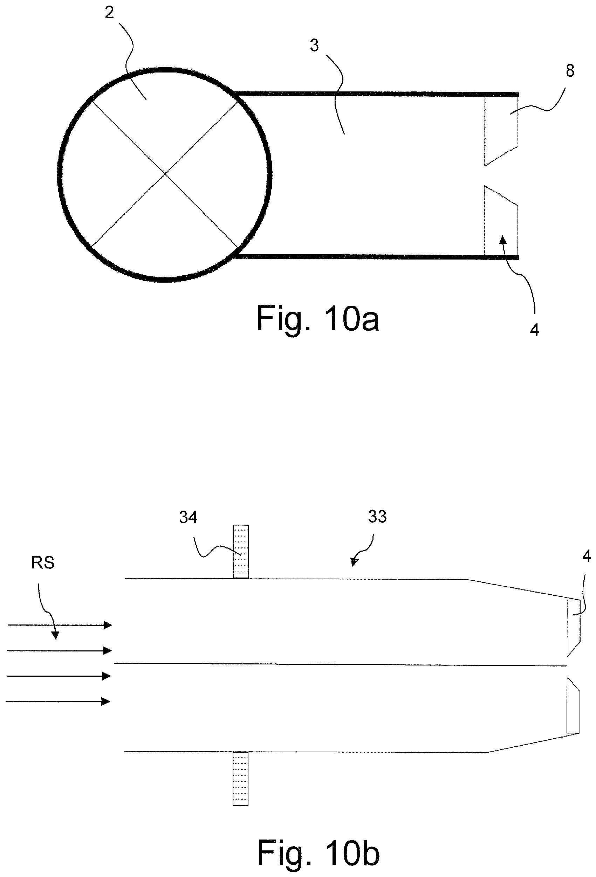

In der **Figur 10a** ist schematisch ein Teil eines erfindungsgemäßen Röntgenanalysegeräts im Bereich der Röntgenquelle 2 dargestellt. Die Röntgenquelle 2 ist hier unmittelbar mit einem strahlformenden Bauteil 3 verbunden, an dessen quellabgewandter Austrittsöffnung eine Röntgenblende 4 mit einkristallinem Blendenkörper 8 angeordnet ist. Ein von der Röntgenblende 4 definierter Röntgenstrahl kann unmittelbar auf eine Probe gerichtet werden, wodurch ein besonders kompakter Aufbau erreicht wird.

**Figur 10b** zeigt einen Kollimator 33, wie er in SCD- und µ-XRD-Messanordnungen bzw. einem entsprechenden erfindungsgemäßen Röntgenanalysegerät eingesetzt werden kann, etwa zwischen Röntgenquelle und Probe. Der Kollimator 33 dient hauptsächlich als Halterung für eine ausgangsseitige Röntgenblende 4 mit einem einkristallinen Blendenkörper (wie etwa in Fig. 2 dargestellt) und ist selbst an einem Halter 34 befestigt. Der Kollimator 33 bzw. die darin enthaltene Röntgenblende 4 dient dazu, den Röntgenstrahl RS zu begrenzen.

**Figur 10c** zeigt eine Ausführungsform eines Kollimators 33 ähnlich der Figur 10b, jedoch mit zwei Röntgenblenden 4a, 4b mit einkristallinem Blendenkörper. Über den Abstand zwischen den beiden Röntgenblenden 4a, 4b und über deren Lochdurchmesser kann die Divergenz des Röntgenstrahls RS eingestellt werden.

### Erfindungsgemäßes Herstellungsverfahren bzw. Nachbearbeitungsverfahren für eine Röntgenblende

Gemäß der Erfindung können streufreie (oder zumindest streuarme) Röntgenblenden, basierend auf einem einkristallinen Blendenkörper, hergestellt werden. Dabei resultiert die geringe parasitäre Streuung nicht allein aus der Verwendung eines einkristallinen Blendenkörpers, sondern auch aus der Führung des Herstellungsverfahrens. Die Erfinder haben festgestellt, dass bestimmte Verfahrensschritte von entscheidender Bedeutung für eine geringe parasitäre Streuung sind, insbesondere ein Nachbehandlungsschritt nach dem Einbringen des durchgehenden Lochs in den Blendenkörper. Die Figuren 11a bis 11d illustrieren das erfindungsgemäße Nachbehandlungsverfahren.

Das im folgenden dargestellte lonenstrahlätzen (hier auch kurz als lonenätzen bezeichnet) kann im Rahmen der erfindungsgemäßen Herstellung einer Röntgenblende mit einkristallinem Blendenkörper eingesetzt werden, oder auch zur erfindungsgemäßen Nachbearbeitung einer vorhandenen Röntgenblende, die beispielsweise bereits in einer Röntgenapparatur eingesetzt wurde und durch den anhaltenden Beschuss mit Röntgenstrahlung und Ablagerungen von Verschmutzungen an Kantenqualität eingebüßt hat.

Zur Nachbehandlung eines bereits mit einem durchgehenden Loch 9 versehenen Blendenkörpers 8 werden gemäß der Erfindung Ionen 21, bevorzugt Ar⁺-Ionen oder andere Edelgasionen, mit hoher kinetischer Energie, typischerweise unter Anwendung einer Beschleunigungsspannung in einem Bereich von 0.1 kV bis 20 kV, auf den Blendenkörper 8 geschossen, vgl. **Figur 11a** ("lonenätzen").

Eine lonenquelle (nicht dargestellt) sendet dazu typischerweise einen lonenstrahl aus, der eine Querschnittsfläche größer als die Querschnittsfläche der ionenquellenzugewandten Öffnung des Blendenkörpers 8 (hier der Austrittsöffnung 11) hat, um eine vollständige Bearbeitung der öffnungszugewandten Blendenkörperoberfläche des Lochs 9 bis zur Kante 10 sicherzustellen. Der lonenstrahl hat meist ein rotationssymmetrisches, gaußverteiltes Intensitätsprofil; die Querschnittsfläche des lonenstrahls ergibt sich aus dem Abfall auf die halbe maximale Intensität. Falls der lonenstrahl eine kleinere Querschnittsfläche als die ionenquellenzugewandte Öffnung der Röntgenblende 4 hat, so kann der lonenstrahl auch relativ zur Röntgenblende 4 bewegt werden (in Fig. 11a in x- und y-Richtung, "parallel" zur Röntgenblende 4), um eine vollständige Bearbeitung sicherzustellen. Durch eine Veränderung des Abstands zwischen lonenquelle und Blendenkörper 8, also eine relative Bewegung in z-Richtung, ist der lonenstrahlquerschnitt einstellbar.

Der lonenstrahl ist bevorzugt parallel zur Symmetrieachse SA der Röntgenblende bzw. senkrecht zur ionenquellenzugewandten Seite (hier Rückseite 14) des Blendenkörpers 8 ausgerichtet, um einen gleichmäßigen Materialabtrag sicherzustellen (Falls nach dem Einbringen des Lochs 9 noch Korrekturen an der Lochform vorgenommen werden müssen, kann auch eine Verschwenkung der lonenquelle vorgenommen werden, so dass der Ionenstrahl gegenüber der Symmetrieachse SA geeignet verkippt ist). Um die Form der Eintrittsöffnung 12 bzw. des Lochs 9 nicht zu verändern, liegt zum einen im Falle einer gaußverteilten lonenstrahlintensität das Intensitätsmaximum bevorzugt auf der Symmetrieachse SA und zum anderen hat der lonenstrahlquerschnitt bevorzugt die gleiche Form wie der Öffnungsquerschnitt im einkristallinen Blendenkörper 8. Bei der Nachbearbeitung kreisrunder Öffnungsquerschnitte im einkristallinen Blendenkörper 8 ist der lonenstrahlquerschnitt demnach bevorzugt ebenfalls kreisrund zu wählen. Falls gewünscht, können über einen Versatz des Intensitätsmaximum (in der xy-Ebene) gegenüber der Symmetrieachse SA auch Formkorrekturen vorgenommen werden.

In der Figur 11a ist der Blendenkörper 8 seitlich mit Klemmen 22 gehalten, die nicht vom lonenstrahl getroffen werden. Die ionenquellenabgewandte Öffnung (hier die Eintrittsöffnung 12) ist nicht verdeckt, so dass abgetragenes Material durch die Öffnung leicht entweichen kann; bevorzugt weist die Öffnung nach unten, so dass die Schwerkraft das Herabfallen von abgetragenem Material begünstigt. Eine gegebenenfalls gewünschte Abdeckstruktur (vgl. Figuren 8a, 8b) wird erst nach Abschluss des lonenätzens angebracht. Absorptionsstrukturen, insbesondere mit Halterung, können wahlweise vor oder (bevorzugt) nach dem lonenätzen angebracht werden.

Es ist auch möglich, den Blendenkörper 8 beim lonenätzen auf eine Auflageplatte (Substrathalterung) 23 aufzulegen, vgl. **Figur 11b****.** Die Auflageplatte 23 hat einen Durchbruch 24, um die ionenquellenabgewandte Öffnung (hier die Eintrittsöffnung 12) nicht zu verdecken.

Nach einer Bearbeitung von der Rückseite 14, wie etwa in Figur 11b gezeigt, kann sich noch eine Bearbeitung von der Vorderseite 13 anschließen, wie in **Figur 11c** dargestellt (oder umgekehrt). Dadurch kann die Kante 10 und deren unmittelbare Umgebung noch besser von Verunreinigungen und oberflächennahen Defekten befreit werden. Auch hierfür kann der Blendenkörper 8 auf eine Auflageplatte 23 mit ausreichend großem Durchbruch 24 aufgelegt werden.

Für die Fertigung größerer Stückzahlen von Röntgenblenden kann auch eine gleichzeitige Bearbeitung mehrerer Blendenkörper 8 mit einem entsprechend großen lonenstrahl vorgenommen werden, wie in **Figur 11d** dargestellt. Die Blendenkörper 8 können dazu auf eine gemeinsame Auflageplatte 23 mit mehreren Durchbrüchen 24 angeordnet werden.

Die **Figur 11e** gibt einen Überblick über den Gesamtablauf eines erfindungsgemäßen Herstellungsverfahrens für Röntgenblenden mit einem einkristallinen Blendenkörper 8.

Zunächst wird ein einstückiger, einkristalliner Blendenkörper 8 bereitgestellt, etwa indem ein entsprechendes Stück aus einem Wafer ausgeschnitten wird.

Sodann wird durch ein geeignetes Verfahren, insbesondere kalte Laserablation 26 und/oder Funkenerosion 27, ein durchgehendes Loch 9 in den Blendenkörper 8 eingebracht. Dies geht in einer oberflächennahen Materialschicht 25 mit der Erzeugung von Defekten im einkristallinen Blendenkörper 8 einher (nicht jedoch in tiefergelegenen Schichten, etwa tiefer als 20-40 µm); weiterhin treten gewisse oberflächliche Verschmutzungen sowie eine erhöhte Rauigkeit auf.

Daher wird anschließend eine Reinigung des Blendenkörpers 8 in einem Ultraschallbad 28 vorgenommen, und schließlich wird durch lonenätzen 29 die defektreiche, oberflächennahe Materialschicht 25 im Bereich des Lochs 9 abgetragen und die Rauigkeit insbesondere in einem Wellenlängenbereich von 0.1 nm bis 10µm reduziert. Durch letztere Maßnahme vergrößert sich die strahldefinierende Querschnittsfläche, die durch die Kante 10 definiert wird; der kleinste Lochdurchmesser nimmt von DMᵥₒᵣ zu DM_{nach} typischerweise um ca. 20-200µm, meist um 100µm, zu.

Der Blendenkörper 8 bzw. dessen Kante 10 kann sodann zur Begrenzung eines Röntgenstrahls bei allenfalls sehr geringer parasitärer Röntgenstrahlung eingesetzt werden.

### Experimenteller Nachweis der Wirkung der Erfindung

Im Folgenden werden experimentelle Daten zum parasitären Streuverhalten unterschiedlicher Röntgenblenden, einschließlich einer erfindungsgemäß nachbearbeiteten Röntgenblende basierend auf einem einkristallinen Blendenkörper, präsentiert.

Zur Bestimmung des parasitären Streuverhaltens unterschiedlicher Röntgenblenden wird eine Messanordnung wie in **Figur 12a** dargestellt verwendet. In einen annähernd parallelen Röntgenstrahl RS (Divergenz=0.7mrad, Strahlquerschnitt-Halbwertsbreite=0.25mm) mit gaußförmiger Intensitätsverteilung wird die zu untersuchende Blende 30 sukzessiv mit einer Schrittweite von 10µm in den Röntgenstrahl RS gefahren. Dabei kann das parasitäre Streusignal (vgl. die parasitäre Streustrahlung 32) der Kante 10 bei einem Streuwinkel größer als 1 mrad am Detektor 6 um den Primärstrahlfänger 7 gemessen werden.

Zur Bestimmung des Anteils des durch die Blende 30 tretenden Lichts gemessen an der Gesamtintensität des Röntgenstrahls (Intₚₐₛₛ/Intₜₒₜₐₗ), wird ein Stück glasartiger Kohlenstoff 31 als Referenzmaterial verwendet. Es handelt sich hierbei um ein in der Wissenschaft bekanntes, stabiles und reproduzierbar streuendes Material. Trifft die Gesamtintensität der Röntgenstrahlung (Blende 30 ist dabei vollständig aus dem Strahlengang entfernt) auf den glasartigen Kohlenstoff 31, erzeugt dieser eine charakteristische Streuintensität Intₜₒₜₐₗ. Diese dient als Referenzwert bei der Bestimmung unbekannter Photonenflüsse.

Zur Bestimmung der durch die Blende 30 tretenden Röntgenlichtintensität wird der glasartige Kohlenstoff 31 nach jedem gefahrenen Schritt der Blende 30 und der Messung der parasitären Streuintensität 32 der Kante 10 in den Strahlengang positioniert. Der glasartige Kohlenstoff 31 erzeugt daraufhin eine Streuintensität Int_{Pass}, woraus sich Intₚₐₛₛ/Intₜₒₜₐₗ berechnen lässt. Das Verhältnis Intpₐₛₛ/Intₜₒₜₐₗ entspricht dabei unmittelbar dem Anteil des durch die Blende 30 tretenden Röntgenlichts gemessen an der gesamten, eingestrahlten Röntgenlichtintensität (bei vollständig aus dem Strahlengang entfernter Blende 30). Im Gegensatz dazu wird von der am Detektor 6 aufgenommenen, von der Kante 10 erzeugten parasitären Streuintensität 32 die Luftstreuung und der Dunkelstrom des Detektors 6 abgezogen. Anschließend wird diese parasitäre Streuintensität auf eine für den Messaufbau typische Gesamtintensität normiert, um Messungen von verschiedenen Blenden 30 vergleichbar zu machen.

In der **Figur 12b** ist dargestellt die normierte parasitäre Streuintensität (linear nach oben aufgetragen, in beliebigen Einheiten, bE) bei verschiedenen Anteilen Intₚₐₛₛ/Intₜₒₜ des durch die Blende 30 getretenen Röntgenlichts (linear nach rechts aufgetragen, zwischen 0 und 1), für verschiedene Blenden:
- Eine handelsübliche polykristalline Röntgenblende aus Platin/Iridium; kreisrunder Lochquerschnitt mit 750µm Durchmesser; α=45, β=90°; (Symbol ▲);
- Eine Röntgenblende mit einkristallinem Blendenkörper aus Germanium, hergestellt durch kalte Laserablation, ohne Nachbehandlung durch lonenätzen; kreisrunder Lochquerschnitt mit 450µm Durchmesser; α=15°, β=90°; (Symbol ◆);
- Eine Röntgenblende mit einkristallinem Blendenkörper aus Germanium, hergestellt durch kalte Laserablation, mit Nachbehandlung durch lonenätzen; kreisrunder Lochquerschnitt mit 550µm Durchmesser; α=15°; β=90°; (Symbol X).

Je Blende wurden an unterschiedlichen Kantenpositionen insgesamt zwei Messdurchgänge durchgeführt, die jeweils beide in Fig. 12b dargestellt sind.

Zu Beginn der jeweiligen Messdurchgänge tritt kein Röntgenlicht durch die Öffnung (Intpₐₛₛ/Intₜₒₜₐₗ=0), was sich im weiteren Verlauf schrittweise ändert (Intpass/Intₜₒₜₐₗ>0). Mit jedem Schritt treffen mehr Röntgenphotonen auf die strahlformende Kante und werden je nach Blende unterschiedlich stark an dieser parasitär gestreut. Bei Intₚₐₛₛ/Intₜₒₜₐₗ=0.5 trifft die maximale Photonenanzahl auf die Kante und erzeugt das größte Streusignal; hier kann das Streuverhalten der unterschiedlichen Blenden gut verglichen werden. Im weiteren Verlauf nimmt die parasitäre Streuung gemäß der gaußverteilten Strahlungsintensität ab.

Wie aus Fig. 12b bei Intₚₐₛₛ/Intₜₒₜₐₗ=0,5 ersichtlich, kann mit einer einkristallinen Röntgenblende ohne lonenätzen gegenüber einer herkömmlichen polykristallinen Blende bereits eine Reduktion der parasitären Streuung von etwa 150-200 bE auf etwa 100 bE, also etwa die Hälfte, erreicht werden. Wird die einkristalline Röntgenblende zusätzlich einer Nachbehandlung durch lonenätzen unterzogen, so sinkt die parasitäre Streuung so stark ab, dass die Messungenauigkeit erreicht wird; bei Intₚₐₛₛ/Intₜₒₜₐₗ=0,5 wurde experimentell noch 4 bE bestimmt, also etwa ein Fünfzigstel des Werts der polykristallinen Blende. Dies lässt auf eine hohe Güte und damit Streufreiheit (bzw. sehr geringe parasitäre Streuung) der erfindungsgemäß hergestellten Blende schließen.

In rasterelektronenmikroskopischen Aufnahmen hat sich gezeigt, dass bei Röntgenblenden mit einkristallinem Blendenkörper durch lonenätzen die Oberflächenrauigkeit deutlich verringert und die Kantenschärfe dadurch erhöht werden kann. In **Figur 12c** ist dazu jeweils eine Rückseite eines einkristallinen Blendenkörpers im Bereich des Lochs vor dem lonenätzen (links) und nach dem lonenätzen (rechts) dargestellt. **Figur 12d** zeigt einen vergrößerten Ausschnitt der jeweiligen Kante des Lochs, wiederum vor dem lonenätzen (links) und nach dem lonenätzen (rechts).

## Patentansprüche

1. Verfahren zur Herstellung einer Röntgenblende (4; 4a, 4b), mit folgenden Schritten:
a) Einbringen eines durchgehenden Lochs (9) in einen einkristallinen Blendenkörper (8), so dass der einkristalline Blendenkörper (8) eine umlaufende, durchgehende Kante (10) zur Begrenzung eines Röntgenstrahls (RS) ausbildet, von der ausgehend sich das Loch (9) in einem ersten Bereich (B1) in Richtung einer Austrittsöffnung (11) trichterförmig erweitert, wobei zum Einbringen des Loches (9) Funkenerosion (17) und/oder kalte Laserablation (26) eingesetzt wird,
b) Abtragen von Material von der Oberfläche des Blendenkörpers (8) zumindest im Bereich der Kante (10) und des ersten Bereichs (B1) durch Ionenstrahlätzen (29).

2. Verfahren nach Anspruch 1, **dadurch gekennzeichnet, dass** das Loch (9) in Schritt a) zumindest im Bereich der Kante (10) und des ersten Bereichs (B1) mit einem runden, insbesondere kreisrunden Querschnitt gefertigt wird.

3. Verfahren nach Anspruch 2, **dadurch gekennzeichnet, dass** in Schritt b) ein lonenstrahl mit kreisrundem Querschnitt und gaußverteilter Intensität eingesetzt wird, dessen Intensitätsmaximum auf einer Symmetrieachse (SA) einer strahldefinierenden Querschnittsfläche des Lochs (9) des einkristallinen Blendenkörpers (8) liegt.

4. Verfahren nach einem der Ansprüche 1 bis 3, **dadurch gekennzeichnet, dass** in Schritt b) eine einer lonenquelle abgewandte Öffnung, nämlich eine Eintrittsöffnung (12) oder die Austrittsöffnung (11) der Röntgenblende (4; 4a 4b), freigehalten wird.

5. Verfahren nach einem der Ansprüche 1 bis 4, **dadurch gekennzeichnet, dass** in Schritt a) das Loch (9) in den einkristallinen Blendenkörper (8) so eingebracht wird, dass ausgehend von der Kante (10) sich das Loch (9) in einem zweiten Bereich (B2) in Richtung einer Eintrittsöffnung (12) der Röntgenblende (4; 4a, 4b) trichterförmig erweitert, und dass in Schritt b) auch im zweiten Bereich (B2) Material von der Oberfläche des Blendenkörpers (8) durch lonenstrahlätzen (29) abgetragen wird.

6. Verfahren zur Nachbearbeitung einer Röntgenblende (4; 4a, 4b), wobei die Röntgenblende (4; 4a, 4b) einen Blendenkörper (8) aufweist, der eine Kante (10) zur Begrenzung eines Röntgenstrahls (RS) ausbildet,
**dadurch gekennzeichnet,**
**dass** Material von der Oberfläche des Blendenkörpers (8) zumindest im Bereich der Kante (10) durch lonenstrahlätzen (29) abgetragen wird, wobei die Röntgenblende (4; 4a, 4b) vor der Materialabtragung durch das lonenstrahlätzen (29) bereits in einem Röntgenanalysegerät (1) eingesetzt wurde.

7. Röntgenanalysegerät (1), umfassend
- eine Röntgenquelle (2), von der ein Röntgenstrahl (RS) ausgeht,
- wenigstens eine Röntgenblende (4; 4a, 4b), die den von der Röntgenquelle (2) ausgehenden Röntgenstrahl (RS) begrenzt,
- eine Probe (5), auf die der von der wenigstens einen Röntgenblende (4; 4a, 4b) begrenzte Röntgenstrahl (RS) gerichtet ist, und
- einen Röntgendetektor (6) zur Detektion einer von der Probe (5) ausgehenden Röntgenstrahlung,
wobei die wenigstens eine Röntgenblende (4; 4a, 4b) von der Probe (5) beabstandet angeordnet ist,
wobei die wenigstens eine Röntgenblende (4; 4a, 4b) einen einkristallinen Blendenkörper (8) mit einem durchgehenden Loch (9) umfasst,
wobei der einkristalline Blendenkörper (8) eine umlaufende, durchgehende, den Röntgenstrahl (RS) begrenzende Kante (10) ausbildet, von der ausgehend sich das Loch (9) in einem ersten Bereich (B1) in Richtung einer Austrittsöffnung (11) der Röntgenblende (4; 4a, 4b) trichterförmig erweitert,
**dadurch gekennzeichnet,**
**dass** die Röntgenblende (4; 4a, 4b) in einem Verfahren nach einem der Ansprüche 1 bis 5 hergestellt ist, so dass bei der Röntgenblende (4; 4a, 4b) in einem Wellenlängenbereich von 10nm bis 1µm zumindest im Bereich der Kante (10) die Rautiefe kleiner als 100nm ist.

8. Röntgenanalysegerät (1) nach Anspruch 7, **dadurch gekennzeichnet, dass** das Loch (9) zumindest im Bereich der Kante (10) und des ersten Bereichs (B1) einen runden, insbesondere kreisrunden Querschnitt aufweist.

9. Röntgenanalysegerät (1) nach einem der Ansprüche 7 oder 8, **dadurch gekennzeichnet, dass** der erste Bereich (B1) kegelstumpfartig ausgebildet ist und einen Öffnungswinkel α zwischen 5° und 60°, bevorzugt zwischen 10° und 30°, besonders bevorzugt zwischen 12° und 18° aufweist.

10. Röntgenanalysegerät (1) nach einem der Ansprüche 7 bis 9, **dadurch gekennzeichnet, dass** ausgehend von der Kante (10) sich das Loch (9) in einem zweiten Bereich (B2) in Richtung einer Eintrittsöffnung (12) der Röntgenblende (4) trichterförmig erweitert.

11. Röntgenanalysegerät (1) nach einem der Ansprüche 7 bis 9, **dadurch gekennzeichnet, dass** die Kante (10) an einer Vorderseite (13) der Röntgenblende (4) ausgebildet ist, so dass die Kante (10) eine Eintrittsöffnung (12) der Röntgenblende (4) mit begrenzt.

12. Röntgenanalysegerät (1) nach einem der Ansprüche 7 bis 11, **dadurch gekennzeichnet, dass** für eine Kantenschärfe der Kante (10) der Röntgenblende (4) gilt: Kantenradius < 5µm.

13. Röntgenanalysegerät (1) nach einem der Ansprüche 7 bis 12, **dadurch gekennzeichnet, dass** der einkristalline Blendenkörper (8) mit einer Absorptionsstruktur (15a-15g) verbunden, insbesondere verklebt, ist,
dass das Material der Absorptionsstruktur (15a-15g) Röntgenstrahlung der Wellenlänge des von der Röntgenquelle (2) ausgehenden Röntgenstrahls (RS) stärker absorbiert als das Material des einkristallinen Blendenkörpers (8), insbesondere wobei das Material der Absorptionsstruktur (15a-15g) Wolfram, Tantal oder Blei ist,
und dass die Absorptionsstruktur (15a-15g) quer zur Ausbreitungsrichtung des von der wenigstens einen Röntgenblende (4; 4a, 4b) begrenzten Röntgenstrahls (RS) gegenüber der Kante (10) zurückgesetzt ist.

14. Röntgenanalysegerät (1) nach einem der Ansprüche 7 bis 13, **dadurch gekennzeichnet, dass** der einkristalline Blendenkörper (8) mit einer Abdeckstruktur (19) verbunden, insbesondere verklebt, ist,
dass das Material der Abdeckstruktur (19) Röntgenstrahlung schwächer absorbiert als das Material des Blendenkörpers (8),
insbesondere wobei das Material der Abdeckstruktur (19) Kapton oder Beryllium ist,
und dass die Abdeckstruktur (19) eine Eintrittsöffnung (12) und/oder die Austrittsöffnung (11) der Röntgenblende (4) überdeckt, insbesondere wobei das Loch (9) durch die Abdeckstruktur (19) gasdicht oder vakuumdicht verschlossen ist.

15. Röntgenanalysegerät (1) nach einem der Ansprüche 7 bis 14, **dadurch gekennzeichnet, dass** das Röntgenanalysegerät (1) zwei von der Probe (5) beabstandet angeordnete Röntgenblenden (4; 4a, 4b) umfasst, die jeweils einen einkristallinen Blendenkörper (8) mit einem durchgehenden Loch (9) umfassen, wobei die einkristallinen Blendenkörper (8) jeweils eine umlaufende, durchgehende, den Röntgenstrahl (RS) begrenzende Kante (10) ausbilden, von der ausgehend sich das Loch (9) des jeweiligen Blendenkörpers (8) in einem ersten Bereich (B1) in Richtung einer Austrittsöffnung (11) der jeweiligen Röntgenblende (4; 4a, 4b) trichterförmig erweitert.

16. Röntgenanalysegerät (1) nach einem der Ansprüche 7 bis 15, **dadurch gekennzeichnet, dass** das Röntgenanalysegerät (1) als eine Kleinwinkelstreuungs-Messanordnung oder eine Einkristallstreuungs-Messanordnung ausgebildet ist, insbesondere mit einem Primärstrahlfänger (7).

17. Röntgenanalysegerät (1) nach einem der Ansprüche 7 bis 15, **dadurch gekennzeichnet, dass** das Röntgenanalysegerät (1) als eine µ-XRD-Messanordnung ausgebildet ist.

## Claims

1. Method of producing an X-ray aperture (4; 4a, 4b) comprising the following steps:
a) introducing a through pinhole (9) into a single crystal aperture body (8), such that the single crystal aperture body (8) forms a circumferential continuous edge (10) for delimiting an X-ray beam (RS), from which edge the pinhole (9) widens like a funnel in a first area (B1) in a direction towards an outlet opening (11), wherein electric discharge machining (17) and/or cold laser ablation (26) is/are used for introducing the pinhole (9),
b) removing material from the surface of the aperture body (8) at least in the area of the edge (10) and of the first area (B1) through ion etching (29).

2. Method according to claim 1, **characterized in that** the pinhole (9) in step a) is produced to have a round, in particular, circular cross-section at least in the area of the edge (10) and of the first area (B1).

3. Method according to claim 2, **characterized in that** an ion beam with circular cross-section and Gaussian distributed intensity is used in step b), the intensity maximum of which is on a symmetry axis (SA) of a beam-defining cross-sectional area of the pinhole (9) of the single crystal aperture body (8).

4. Method according to any one of the claims 1 through 3, **characterized in that** an opening facing away from an ion source, i.e. an inlet opening (12) or the outlet opening (11) of the X-ray aperture (4; 4a, 4b) is kept free in step b).

5. Method according to any one of the claims 1 through 4, **characterized in that** the pinhole (9) is introduced into the single crystal aperture body (8) in step a) in such a fashion that the pinhole (9) widens like a funnel starting from the edge (10) in a second area (B2) towards an inlet opening (12) of the X-ray aperture (4; 4a, 4b) and that in step b) material is also removed from the surface of the aperture body (8) in the second area (B2) by ion etching (29).

6. Method for post-processing an X-ray aperture (4; 4a, 4b), wherein the X-ray aperture (4; 4a, 4b) comprises an aperture body (8) which forms an edge (10) for delimiting an X-ray beam (RS),
**characterized in that**
material is removed from the surface of the aperture body (8) at least in the area of the edge (10) by ion etching (29), wherein the X-ray aperture (4; 4a, 4b) has already been used in an X-ray analysis apparatus (1) prior to material removal by ion etching (29).

7. X-ray analysis apparatus (1) comprising
- an X-ray source (2) from which an X-ray beam (RS) is emitted,
- at least one X-ray aperture (4; 4a, 4b) which delimits the X-ray beam (RS) emitted by the X-ray source (2),
- a sample (5) onto which the X-ray beam (RS), which is delimited by the at least one X-ray aperture (4; 4a, 4b), is directed, and
- an X-ray detector (6) for detecting X-ray radiation emanating from the sample (5),
wherein the at least one X-ray aperture (4; 4a, 4b) is disposed at a separation from the sample (5),
wherein the at least one X-ray aperture (4; 4a, 4b) comprises a single crystal aperture body (8) with a through pinhole (9),
wherein the single crystal aperture body (8) forms a circumferential continuous edge (10) which delimits the X-ray beam (RS) and starting from which the pinhole (9) widens like a funnel in the direction of an outlet opening (11) of the X-ray aperture (4; 4a, 4b) in a first area (B1),
**characterized in that**
the X-ray aperture (4; 4a, 4b) is produced using a method according to one of the claims 1 through 5 such that the roughness depth of the X-ray aperture (4; 4a, 4b) in a wavelength range between 10nm and 1µm is smaller than 100nm at least in the area of the edge (10).

8. X-ray analysis apparatus (1) according to claim 7, **characterized in that** the pinhole (9) has a round, in particular, circular cross-section at least in the area of the edge (10) and of the first area (B1).

9. X-ray analysis apparatus (1) according to any one of the claims 7 or 8, **characterized in that** the first area (B1) is designed like a truncated cone and has an opening angle α of between 5° and 60°, preferably between 10° and 30°, particularly preferred between 12° and 18°.

10. X-ray analysis apparatus (1) according to any one of the claims 7 through 9, **characterized in that**, starting from the edge (10), the pinhole (9) widens like a funnel in a second area (B2) in the direction towards an inlet opening (12) of the X-ray aperture (4).

11. X-ray analysis apparatus (1) according to any one of the claims 7 through 9, **characterized in that** the edge (10) is formed at a front side (13) of the X-ray aperture (4) such that the edge (10) also delimits an inlet opening (12) of the X-ray aperture (4).

12. X-ray analysis apparatus (1) according to any one of the claims 7 through 11, **characterized in that** the following applies to an edge sharpness of the edge (10) of the X-ray aperture (4): edge radius < 5µm.

13. X-ray analysis apparatus (1) according to any one of the claims 7 through 12, **characterized in that** the single crystal aperture body (8) is connected, in particular, glued to an absorption structure (15a-15g),
the material of the absorption structure (15a-15g) absorbs X-ray radiation of a wavelength of the X-ray beam (RS) emitted by the X-ray source (2) to a greater extent than the material of the single crystal aperture body (8), in particular, wherein the material of the absorption structure (15a-15g) is tungsten, tantalum or lead, and the absorption structure (15a-15g) is recessed with respect to the edge (10) in a direction transverse to the direction of propagation of the X-ray beam (RS) which is delimited by the at least one X-ray aperture (4; 4a, 4b).

14. X-ray analysis apparatus (1) according to any one of the claims 7 through 13, **characterized in that** the single crystal aperture body (8) is connected, in particular glued, to a cover structure (19),
the material of the cover structure (19) absorbs X-ray radiation to a lesser extent than the material of the aperture body (8),
in particular, wherein the material of the cover structure (19) is Kapton or beryllium, and the cover structure (19) covers an inlet opening (12) and/or the outlet opening (11) of the X-ray aperture (4), in particular,
wherein the pinhole (9) is closed by the cover structure (19) in a gas-tight or vacuum-tight fashion.

15. X-ray analysis apparatus (1) according to any one of the claims 7 through 14, **characterized in that** the X-ray analysis apparatus (1) comprises two X-ray apertures (4; 4a, 4b) which are disposed at a separation from the sample (5), each comprising a single crystal aperture body (8) with a through pinhole (9), wherein the single crystal aperture bodies (8) each form a circumferential continuous edge (10) that delimits the X-ray beam (RS) and starting from which the pinhole (9) of the respective aperture body (8) widens like a funnel in a first area (B1) towards an outlet opening (11) of the respective X-ray aperture (4; 4a, 4b).

16. X-ray analysis apparatus (1) according to any one of the claims 7 through 15, **characterized in that** the X-ray analysis apparatus (1) is designed as a small angle X-ray scattering measuring arrangement or a single crystal X-ray scattering measuring arrangement, in particular, comprising a primary beam stop (7).

17. X-ray analysis apparatus (1) according to any one of the claims 7 through 15, **characterized in that** the X-ray analysis apparatus (1) is designed as a µ-XRD measuring arrangement.

## Revendications

1. Procédé de fabrication d'un diaphragme à rayons X (4 ; 4a, 4b), comprenant les étapes suivantes :
a) réalisation d'un trou traversant (9) dans un corps de diaphragme monocristallin (8), de façon que le corps de diaphragme monocristallin (8) forme une arête périphérique continue (10) destinée à délimiter un rayon X (RS), à partir de laquelle, dans une première zone (B1), le trou (9) s'élargit en forme d'entonnoir en direction d'une ouverture de sortie (11), le trou (9) étant réalisé par électroérosion (17) et/ou par ablation laser à froid (26),
b) enlèvement de matière de la surface du corps de diaphragme (8) au moins dans la zone de l'arête (10) et de la première zone (B1) par attaque par faisceau d'ions (29).

2. Procédé selon la revendication 1, **caractérisé en ce qu'**à l'étape a), le trou (9) est réalisé avec une section transversale ronde, en particulier circulaire, au moins dans la zone de l'arête (10) et de la première zone (B1).

3. Procédé selon la revendication 2, **caractérisé en ce qu'**à l'étape b), on utilise un faisceau d'ions de section transversale circulaire et d'intensité à distribution gaussienne, dont le maximum d'intensité se situe sur un axe de symétrie (SA) d'une surface de section transversale définissant le faisceau du trou (9) du corps de diaphragme monocristallin (8).

4. Procédé selon l'une des revendications 1 à 3, **caractérisé en ce qu'**à l'étape b), une ouverture éloignée de la source d'ions, à savoir une ouverture d'entrée (12) ou l'ouverture de sortie (11) du diaphragme à rayons X (4 ; 4a, 4b), est maintenue libre.

5. Procédé selon l'une des revendications 1 à 4, **caractérisé en ce qu'**à l'étape a), le trou (9) dans le corps de diaphragme monocristallin (8) est réalisé de façon qu'à partir de l'arête (10), dans une deuxième zone (B2), le trou (9) s'élargisse en forme d'entonnoir en direction d'une ouverture d'entrée (12) du diaphragme à rayons X (4 ; 4a, 4b), et qu'à l'étape b), de la matière est enlevée de la surface du corps de diaphragme (8) par attaque par faisceau d'ions (29) également dans la deuxième zone (B2).

6. Procédé de rectification d'un diaphragme à rayons X (4 ; 4a, 4b), lequel diaphragme à rayons X (4 ; 4a, 4b) présente un corps de diaphragme (8) qui forme une arête (10) destinée à délimiter un rayon X (RS),
**caractérisé en ce**
**que** de la matière est enlevée de la surface du corps de diaphragme (8) par attaque par faisceau d'ions (29) au moins dans la zone de l'arête (10), le diaphragme à rayons X (4 ; 4a, 4b) ayant déjà été utilisé dans un appareil d'analyse à rayons X avant l'enlèvement de matière par l'attaque par faisceau d'ions (29).

7. Appareil d'analyse à rayons X (1), comprenant
- une source de rayons X (2) dont provient un rayon X (RS),
- au moins un diaphragme à rayons X (4 ; 4a, 4b) qui délimite le rayon X (RS) provenant de la source de rayons X (2),
- un échantillon (5) sur lequel le rayon X (RS) délimité par ledit au moins un diaphragme à rayons X (4 ; 4a, 4b) est dirigé, et
- un détecteur de rayons X (6) pour détecter un rayonnement X provenant de l'échantillon (5),
ledit au moins un diaphragme à rayons X (4 ; 4a, 4b) étant disposé à distance de l'échantillon (5),
ledit au moins un diaphragme à rayons X (4 ; 4a, 4b) comprenant un corps de diaphragme monocristallin (8) muni d'un trou traversant (9),
le corps de diaphragme monocristallin (8) formant une arête (10) périphérique continue, délimitant le rayon X (RS), à partir de laquelle, dans une première zone (B1), le trou (9) s'étend en forme d'entonnoir en direction d'une ouverture de sortie (11) du diaphragme à rayons X (4 ; 4a, 4b),
**caractérisé en ce**
**que** le diaphragme à rayons X (4 ; 4a, 4b) est fabriqué dans un procédé selon l'une des revendications 1 à 5, de façon que, dans le cas du diaphragme à rayons X (4 ; 4a, 4b) dans une gamme de longueurs d'onde de 10 nm à 1 µm, la profondeur de rugosité soit inférieure à 100 nm au moins dans la zone de l'arête (10).

8. Appareil d'analyse à rayons X (1) selon la revendication 7, **caractérisé en ce que** le trou (9) présente une section transversale ronde, en particulier circulaire, au moins dans la zone de l'arête (10) et de la première zone (B1).

9. Appareil d'analyse à rayons X (1) selon l'une des revendications 7 ou 8, **caractérisé en ce que** la première zone (B1) est réalisée en forme de tronc de cône et présente un angle d'ouverture α compris entre 5° et 60°, de préférence entre 10° et 30°, particulièrement de préférence entre 12° et 18°.

10. Appareil d'analyse à rayons X (1) selon l'une des revendications 7 à 9, **caractérisé en ce qu'**à partir de l'arête (10), dans une deuxième zone (B2), le trou (9) s'élargit en forme d'entonnoir en direction d'une ouverture d'entrée (12) du diaphragme à rayons X (4).

11. Appareil d'analyse à rayons X (1) selon l'une des revendications 7 à 9, **caractérisé en ce que** l'arête (10) est formée sur une face avant (13) du diaphragme à rayons X (4), de sorte que l'arête (10) délimite également une ouverture d'entrée (12) du diaphragme à rayons X (4).

12. Appareil d'analyse à rayons X (1) selon l'une des revendications 7 à 11, **caractérisé en ce que** pour une acuité d'arête de l'arête (10) du diaphragme à rayons X (4), il s'applique : rayon d'arête < 5 µm.

13. Appareil d'analyse à rayons X (1) selon l'une des revendications 7 à 12, **caractérisé en ce**
**que** le corps de diaphragme monocristallin (8) est relié, en particulier collé, à une structure d'absorption (15a-15g), que le matériau de la structure d'absorption (15a-15g) absorbe plus fortement un rayonnement X de la longueur d'onde du rayon X (RS) provenant de la source de rayons X que le matériau du corps de diaphragme monocristallin (8), en particulier le matériau de la structure d'absorption (15a-15g) étant du tungstène, du tantale ou du plomb,
et **que** la structure d'absorption (15a-15g) est en retrait par rapport à l'arête (10) transversalement à la direction de propagation du rayon X (RS) délimité par ledit au moins un diaphragme à rayons X (4 ; 4a, 4b).

14. Appareil d'analyse à rayons X (1) selon l'une des revendications 7 à 13, **caractérisé en ce**
**que** le corps de diaphragme monocristallin (8) est relié, en particulier collé, à une structure de recouvrement (19),
**que** le matériau de la structure de recouvrement (19) absorbe le rayonnement X plus faiblement que le matériau du corps de diaphragme (8),
en particulier le matériau de la structure de recouvrement (19) étant du kapton ou du béryllium,
et **que** la structure de recouvrement (19) recouvre une ouverture d'entrée (12) et/ou l'ouverture de sortie (11) du diaphragme à rayons X (4), en particulier le trou (9) étant fermé de manière étanche aux gaz ou au vide par la structure de recouvrement (19).

15. Appareil d'analyse à rayons X (1) selon l'une des revendications 7 à 14, **caractérisé en ce que** l'appareil d'analyse à rayons X (1) comprend deux diaphragmes à rayons X (4 ; 4a, 4b) disposés à distance de l'échantillon (5), qui comprennent chacun un corps de diaphragme monocristallin (8) muni d'un trou traversant (9), les corps de diaphragme monocristallins (8) formant chaque fois une arête (10) périphérique continue, délimitant le rayon X (RS), à partir de laquelle, dans une première zone (B1), le trou (9) du corps de diaphragme (8) respectif s'étend en forme d'entonnoir en direction d'une ouverture de sortie (11) du diaphragme à rayons X (4 ; 4a, 4b) respectif.

16. Appareil d'analyse à rayons X (1) selon l'une des revendications 7 à 15, **caractérisé en ce que** l'appareil d'analyse à rayons X (1) est réalisé sous la forme d'un dispositif de mesure de la diffusion aux petits angles ou un dispositif de mesure de la diffusion par un monocristal, en particulier avec un piège à faisceau primaire (7).

17. Appareil d'analyse à rayons X (1) selon l'une des revendications 7 à 15, **caractérisé en ce que** l'appareil d'analyse à rayons X (1) est réalisé sous la forme d'un dispositif de mesure µ-XRD.
